# EUROPEAN PATENT SPECIFICATION

(11) **EP 2 839 872 B1**
(45) Date of publication and mention of the grant of the patent: **07.03.2018**
(21) Application number: 14003110.5
(22) Date of filing: 12.05.2004
(51) Int. Cl.: B01F 15/02, A61B 17/88, A61F 2/30, A61F 2/46, B01F 11/00, B01F 13/00, B01F 13/06

(54) **Delivery gun for dispensing bone cement from a cartridge, the gun having a multi-link linkage capable of dispensing the cement at different flow rates**
Spritzpistole zum Austragen von Knochenzement, die das Austragen mit zwei verschiedenen Geschwindigkeiten ermöglicht.
Pistolet d'injection de ciment osseux, capable d'injecter à deux vitesses différentes.

(30) Priority: 12.05.2003 US 469651 P; 18.11.2003 US 520877 P
(43) Date of publication of application: 25.02.2015
(62) Divisional of application: 13003233.7
(73) Proprietor: Stryker Corporation, Kalamazoo, MI 49002 (US)
(72) Inventor: Henniges, Bruce D., Galesburg, MI 49053 (US); Tague, Christopher Matthew, Delton, MI 49046-9761 (US); Coffeen, Jared Paul, Paw Paw, 49079 (US); Brockman, Christopher S., Kalamazoo, MI 49008 (US); Proulx, Marshall K., Portage, MI 49002 (US)
(74) Representative: Röthinger, Rainer

(56) References cited:
- EP-A1- 0 621 084
- US-A- 3 029 653
- US-A- 5 052 243

## Description

### FIELD OF THE INVENTION

The present invention generally relates to a bone cement mixing and delivery system. More specifically, the present invention relates to a delivery gun for discharging bone cement from a mixing cartridge into an anatomical site of a patient. The mixing cartridge is provided for receiving liquid and powder components of the bone cement to be mixed by a mixing device.

### BACKGROUND OF THE INVENTION

Bone cement mixing and delivery systems are well known for mixing liquid and powder components of bone cement and delivering the prepared bone cement to an anatomical site during various surgical procedures. Bone cement is particularly useful in orthopedic procedures in which a prosthetic device is fixed to a bone or joint structure to improve the strength, rigidity, and movement of the structure. In a total hip arthroplasty (THA) procedure, in which a hip joint is replaced with a prosthetic device, bone cement is used to fix the prosthetic device in place in a medullary canal of a femur.

Typically, the bone cement is prepared in a mixing cartridge. The mixing cartridge includes a cylinder having proximal and distal ends with a mixing chamber defined between the ends. The mixing cartridge further includes a cap covering the proximal end of the cylinder and a piston disposed in the distal end of the cylinder such that the mixing chamber is further defined between the cap and the piston. The piston may be releasably secured in a locked position in the cylinder by a cotter pin. The cap supports a mixing device, i.e., a mixing shaft and blade, for mixing the liquid and powder components of the bone cement in the mixing chamber.

Once the bone cement is mixed, the mixing cartridge is prepared for inserting into a delivery gun to discharge the bone cement. This may include disengaging the mixing shaft and coupling a nozzle to the cap to provide a discharge point for the bone cement. At the same time, the piston is released from the locked position in the distal end of the cylinder by pulling the cotter pin. This allows the piston to be driven by the delivery gun through the mixing chamber to discharge the bone cement from the nozzle. An alternative solution for securing and releasing the piston is shown in United States Patent No. 5,328,262 to Lidgren et al.

In Lidgren et al., the piston is releasably secured in the locked position in the distal end of the cylinder by a gripping portion in the form of a flange, which extends along only a portion of an inner periphery of the cylinder. The piston in Lidgren et al. has a corresponding gripping portion in the form of an outwardly directed lip that protrudes behind the flange. The lip defines a groove with an outer surface of the piston to receive the flange. To release the piston from the locked position, the flange is rotated through the groove until the flange has been rotated past the lip. Lidgren et al. discloses a base that is used to secure the piston from rotation while a user rotates the cylinder relative to the piston to release the piston from the locked position. This method of releasing the piston from the locked position, much like pulling the cotter pin, requires additional manipulation by a user.

Once the piston is released from the locked position, the mixing cartridge is inserted into the delivery gun. A typical delivery gun includes a ram disk that engages the piston and drives the piston through the mixing chamber to discharge the bone cement from the nozzle. The delivery gun includes a cradle for supporting the mixing cartridge and a casing for supporting a drive rod that engages the ram disk and advances the ram disk to drive the piston. The drive rod includes a plurality of teeth and a pawl member engages the teeth to advance the drive rod. A trigger supports the pawl member and the casing rotatably supports the trigger. Actuation of the trigger relative to the casing urges the pawl member against the teeth to advance the drive rod.

An example of such a delivery gun is illustrated in United States Patent No. 5,431,654 to Nic. In the '654 patent to Nic, two pawl members are used to independently advance the drive rod and the ram disk. The pawl members provide high speed/low force and low speed/high force advancement of the drive rod. A switch is used to select between the speeds. When high speed is selected, both pawl members engage the drive rod, while only the high-speed pawl member actually advances the drive rod. When low speed is selected, the high-speed pawl member is isolated from the teeth such that only the low speed pawl member engages the teeth to advance the drive rod. However, in Nic, the trigger directly supports each of the pawl members which results in a low mechanical advantage to advance the drive rod and ram disk.

The European Patent Application No. 0 621 084 A1 discloses a manually operated actuating device comprising a fixed handle and a movable actuating lever which is connected to an actuatable apparatus part via further levers.

Further prior art is described in the patent document US 5 052 243 A which discloses a hand power tool mechanism for bone cement caulking guns.

### BRIEF SUMMARY OF THE INVENTION

Only as an example and useful for understanding the invention, a mixing cartridge for receiving liquid and powder components of bone cement to be mixed for medical use is described. The mixing cartridge comprises a cylinder having proximal and distal ends with a mixing chamber defined therebetween. The cylinder includes a cylinder wall extending between the ends about a longitudinal axis of the cylinder. A piston is disposed in the cylinder at the distal end such that the mixing chamber is further defined between the proximal end and the piston. A locking member is coupled to the piston to lock the piston in the distal end. The locking member includes a male portion engaging a female portion in the cylinder wall to place the piston in a locked position at the distal end of the cylinder. The locking member includes a resilient portion for biasing the male portion into mating engagement with the female portion. The piston remains in the locked position at the distal end of the cylinder while mixing the liquid and powder components.

One advantage of the mixing cartridge is the conveniently positioned locking member used to lock the piston in the distal end. By using the resilient portion to bias the male portion into mating engagement with the female portion, a user can easily release the piston from the locked position by either manually or mechanically acting against the bias of the resilient portion to disengage the male and female portions.

According to the invention a delivery gun is provided for discharging the bone cement from the cartridge once the bone cement is prepared. The delivery gun comprises a casing for supporting the cartridge. A drive mechanism is supported by the casing and advanceable relative to the casing to force the bone cement from the cartridge. The casing pivotally supports a trigger operatively connected to the drive mechanism to advance the drive mechanism upon actuation of the trigger to force the bone cement from the cartridge. A linkage system works in conjunction with the trigger to advance the drive mechanism. The linkage system comprises a first link pivotally connected to the casing and a second link interconnecting the first link and the trigger such that actuating the trigger moves the second link and the first link to advance the drive mechanism.

An advantage of the delivery gun is the use of the linkage system to increase the mechanical advantage needed to successfully advance the drive mechanism and force the bone cement from the cartridge while minimizing fatigue to a user of the delivery gun.

In one aspect of the delivery gun, the drive mechanism includes a drive rod and gripper plates to advance the drive rod. The gripper plates frictionally engage the drive rod to advance the drive rod when the trigger is actuated. In one embodiment, the gripper plates include mating pegs and notches to align adjacent gripper plates. In another embodiment, the gripper plates are coated to increase lubricity and corrosion resistance thereof.

In another aspect of the delivery gun, the drive mechanism includes a drive rod and first and second pawl members to advance the drive rod. In one embodiment, the second pawl member is movable into engagement with teeth on the drive rod for high-speed advancement of the drive rod and out from engagement with the teeth for low-speed advancement. During low-speed advancement, only the first pawl member engages the teeth to advance the drive rod. During high-speed advancement, both pawl members engage the teeth, but only the second pawl member works to advance the drive rod.

Also a bone cement mixing and delivery system can be provided. The mixing and delivery system includes the exemplary cartridge and the delivery gun. In this aspect, the locking member includes a release button to release the piston from the locked position. At the same time, the delivery gun includes a release mechanism integrated into the drive mechanism to engage the release button. When the cartridge is placed into the cradle of the delivery gun, the drive mechanism is advanced and the release mechanism engages the release button to release the piston from the locked position. This configuration reduces the number of steps typically associated with releasing the piston. By incorporating the release mechanism into the drive mechanism, when the drive mechanism is advanced, the piston is automatically released.

Only exemplarily and useful for understanding the invention, a bone cement loading system for receiving the liquid and powder components of the bone cement can be provided. The loading system includes the cylinder with the piston locked in the distal end. A base defining a cavity is provided for receiving and securing the distal end of the cylinder. A funnel is provided for coupling to the proximal end of the cylinder to channel the powder component of the bone cement into the mixing chamber. The funnel has a proximal end with an oblong oval-shaped periphery to facilitate loading of the powder component of the bone cement into the mixing chamber and a distal end with a circular periphery for snugly fitting into the proximal end of the cylinder. One particular advantage to this loading system is the use of the oblong oval-shaped funnel. The shape of the funnel reduces any mess typically associated with filling the mixing chamber with powder.

Furthermore, only as an example and useful for understanding the invention, a bone cement mixing system comprising the mixing cartridge and a mixing shaft and blade can be provided. The blade is coupled to the mixing shaft and disposed in the mixing chamber for rotating with the mixing shaft about the longitudinal axis to mix the liquid and powder components of the bone cement. The blade includes a center hub coupled to the mixing shaft and an outer ring extending from the center hub. The outer ring forms an acute angle with the longitudinal axis of between twenty and seventy degrees to ensure adequate mixing of the bone cement in the mixing chamber.

Also as an example and useful for understanding the invention, a method of mixing the liquid and powder components of the bone cement in the mixing chamber can be provided. The method includes using a rotary power tool connected to a portion of the mixing shaft extending outside of the mixing chamber to mix the liquid and powder components of the bone cement. The blade is disposed in the mixing chamber while being operatively connected to the portion of the mixing shaft extending outside of the mixing chamber. In the method, the rotary power tool is first connected to the portion of the mixing shaft extending outside of the mixing chamber. Then the rotary power tool is actuated to rotate the blade and mix the liquid and powder components of the bone cement. At the same time, the rotary power tool is axially displaced relative to the mixing cartridge to completely mix the liquid and powder components of the bone cement. Once mixing is complete, the operative connection between the blade and the portion of the mixing shaft extending outside of the mixing chamber is removed.

### BRIEF DESCRIPTION OF THE SEVERAL VIEWS OF THE DRAWINGS

Advantages of the present invention will be readily appreciated as the same becomes better understood by reference to the following detailed description when considered in connection with the accompanying drawings wherein:
Figure 1 is an exploded perspective view of an exemplary mixing cartridge in combination with an exemplary mixing shaft and blade;
Figure 2 is an assembled perspective view of the exemplary mixing cartridge with the exemplary mixing shaft and blade supported therein;
Figure 3 is an exploded perspective view of a cap of the exemplary mixing cartridge;
Figure 4 is a cross-sectional view of the cap of Figure 3 and a partial cross-sectional view of a cylinder of the exemplary mixing cartridge to illustrate fitting of the cap to the cylinder;
Figure 5 is an exploded perspective view of the cap and the exemplary mixing shaft and blade;
Figure 6 is an assembled perspective view of the cap with the exemplary mixing shaft and blade supported therein;
Figure 7 is a perspective view of the blade;
Figure 7A is a side elevational view of the blade of Fig. 7;
Figures 8-8A and 9 are perspective views of alternative blades;
Figure 10 is an exploded perspective view of the exemplary mixing shaft and a latch rod;
Figure 11 is an elevational end view of the exemplary mixing shaft and latch rod of Fig. 10;
Figure 12 is a cross-sectional view of the exemplary mixing shaft and latch rod of Figs. 10 and 11;
Figure 13 is an exploded perspective view of a release latch coupling the exemplary mixing shaft and latch rod;
Figures 14A-14C illustrate the release of the exemplary blade from the exemplary mixing shaft;
Figure 15 is an exploded perspective view of a piston of the exemplary mixing cartridge;
Figure 16 is a cross-sectional view of the piston of Fig. 15;
Figure 17 is a perspective view of an alternative piston of the exemplary mixing cartridge;
Figure 18 is a top view of the alternative piston of Fig. 17;
Figure 19 is an exploded perspective view of the cap and a nozzle;
Figure 20 is an assembled perspective view of the cap and nozzle;
Figure 21 is a blown-up view of a locking mechanism of the cap and nozzle;
Figures 22-23 are perspective views of the nozzle;
Figure 24 is a perspective view of a delivery gun of the present invention illustrating a linkage system of the delivery gun;
Figures 24A-24B illustrate alternative linkage systems of the present invention;
Figure 25 is an elevational view illustrating release of a locking member securing the piston;
Figure 26 is a partial perspective view of an alternative linkage system and drive mechanism of the delivery gun;
Figure 27 is a partial perspective view of the alternative linkage system and drive mechanism of Fig. 26 employing a striker to prevent freeze-up of the drive mechanism;
Figure 28 is an elevational view of a second alternative embodiment of the linkage system and drive mechanism of the delivery gun in a low-speed position;
Figure 29 is a perspective view of the second alternative embodiment of the linkage system and drive mechanism in the low-speed position;
Figure 30 is an elevational view of the second alternative embodiment of the linkage system and drive mechanism in a high-speed position;
Figure 31 is a perspective view of the second alternative embodiment of the linkage system and drive mechanism in the high-speed position;
Figure 32 is an exploded view of a cylinder of the exemplary mixing cartridge and a base and funnel used to fill the cylinder with components of bone cement; and
Figures 33-42 illustrate various steps associated with the present invention.

### DETAILED DESCRIPTION OF THE INVENTION

Referring to the Figures, wherein like numerals indicate like or corresponding parts throughout the several views, a bone cement mixing and delivery system is generally shown. The bone cement mixing and delivery system comprises an exemplary mixing cartridge 100 for receiving liquid monomer and powdered copolymer components of bone cement to be mixed, an exemplary mixing device (mixing shaft 150 and blade 152) for mixing the components, and a delivery device according to the invention, e.g., a delivery gun 500, for discharging the bone cement from the mixing cartridge 100 into an anatomical site (not shown). An exemplary use for the bone cement is to secure a prosthetic device used to replace a joint structure such as in a total hip arthroplasty (THA) procedure.

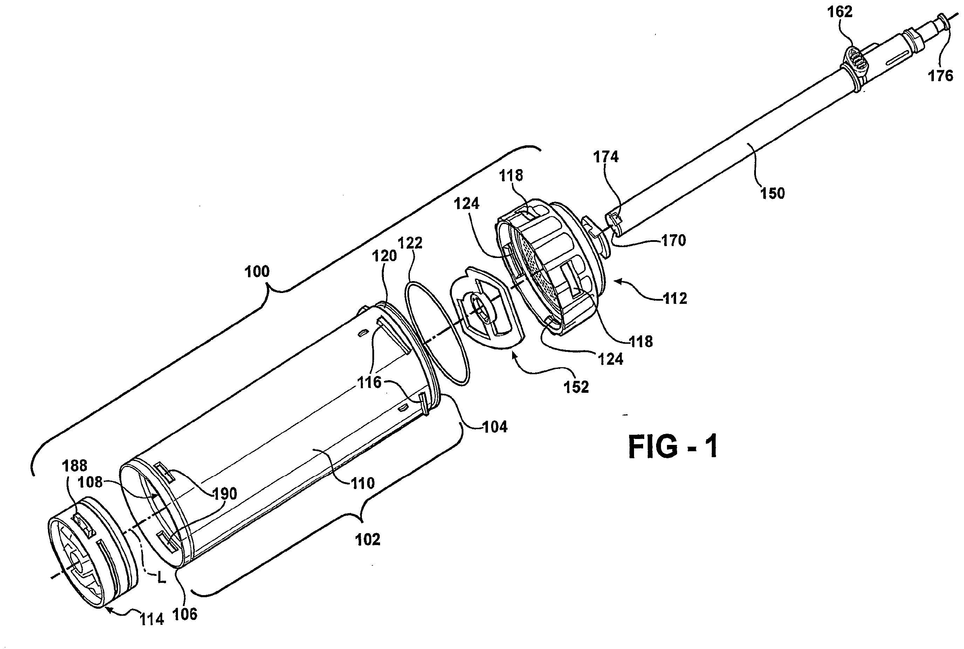

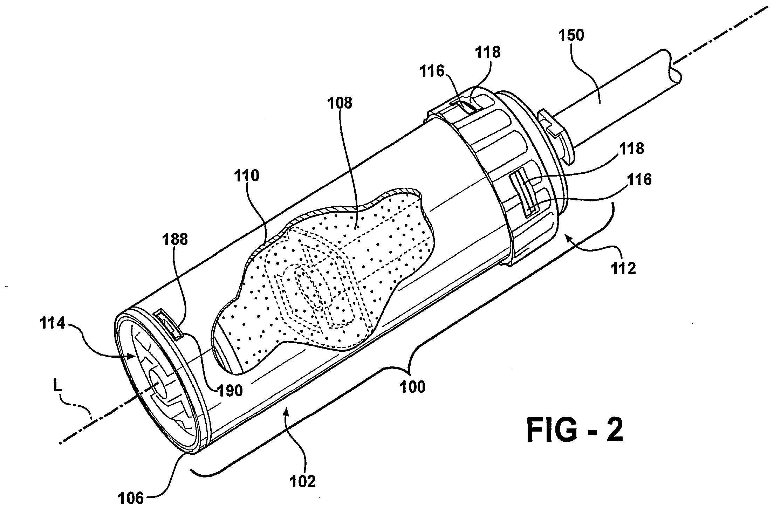

Referring exemplarily to FIGS. 1 and 2 which are useful for understanding the invention, the bone cement mixing system comprises the mixing cartridge 100 in combination with the mixing shaft 150 and blade 152 used to mix the components of the bone cement in the mixing cartridge 100. The mixing cartridge 100 includes a cylinder 102 having proximal 104 and distal 106 ends. A mixing chamber 108 is defined between the ends 104, 106. The cylinder 102 includes a cylinder wall 110 extending between the ends 104, 106, about a longitudinal axis L. A cap 112 is coupled to the cylinder 102 at the proximal end 104 and a piston 114 is disposed in the cylinder 102 at the distal end 106 such that the mixing chamber 108 is further defined between the cap 112 and the piston 114. The components of the bone cement are placed in the mixing chamber 108 and mixed by the mixing shaft 150 and blade152, as will be described further below.

In this embodiment, the cylinder 102 has locking strips 116 disposed on the cylinder wall 110 at the proximal end 104 to insert into locking slots 118 on the cap 112. Each of the locking strips 116 include a straight portion lying perpendicular relative to the longitudinal axis L and an angled portion lying at an angle relative to the straight portion. As should be appreciated, the locking strips 116 and locking slots 118 could be reversed, i.e., the locking strips 116 positioned on the cap 112 and the locking slots 118 defined in the cylinderwall 110. The locking strips 116 and locking slots 118 are configured to provide quick locking of the cap 112 onto the cylinder 102 with a one-quarter turn of the cap 112. Those of ordinary skill in the art will appreciate that numerous methods are available for connecting the cap 112 to the cylinder 102, such as mating threads, snap-fit connections, etc. A groove 120 is formed in the cylinder 102 at the proximal end 104 to seat an o-ring seal 122. The o-ring seal 122 assists in sealing the cap 112 to the cylinder 102.

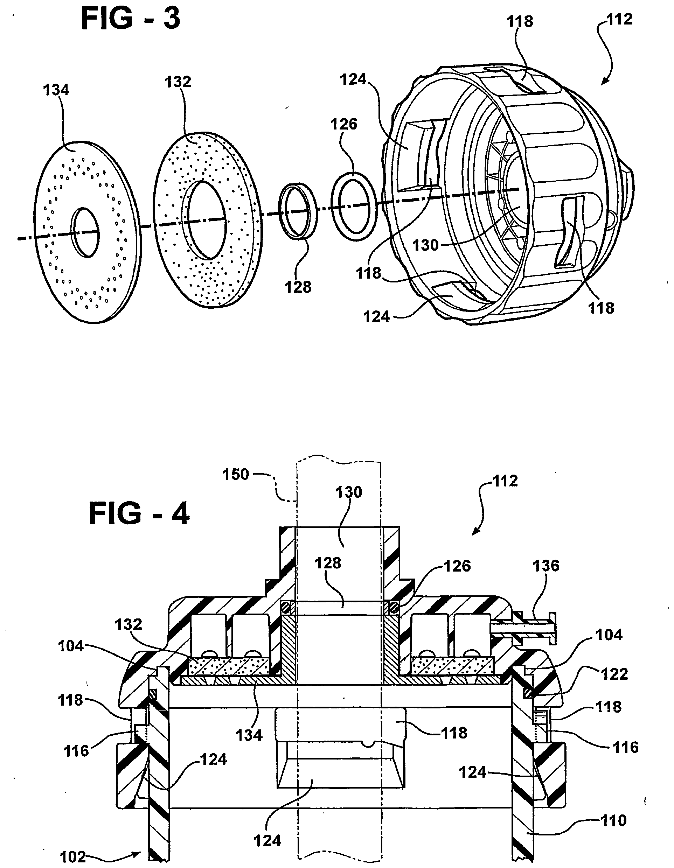

Referring exemplarily to FIGS. 3-4 which are useful for understanding the invention, the cap 112 includes radially inwardly protruding ramps 124 that lead into the locking slots 118 to facilitate the fit with the locking strips 116 on the cylinder wall 110. When first placing the cap 112 on the cylinder 102, the locking strips 116 are positioned between the ramps 124. As the cap 112 is rotated, the ramps 124 cam the locking strips 116 proximally to urge the proximal end 104 of the cylinder 102 into a sealed relationship with the cap 112, as shown in FIG. 4 (only a portion of the cylinder wall 110 with two locking strips 116 is shown in FIG. 4 for illustrative purposes). In the preferred embodiment, there are four locking strips 116 and four locking slots 118 to facilitate the sealed relationship between the cap 112 and the cylinder 102.

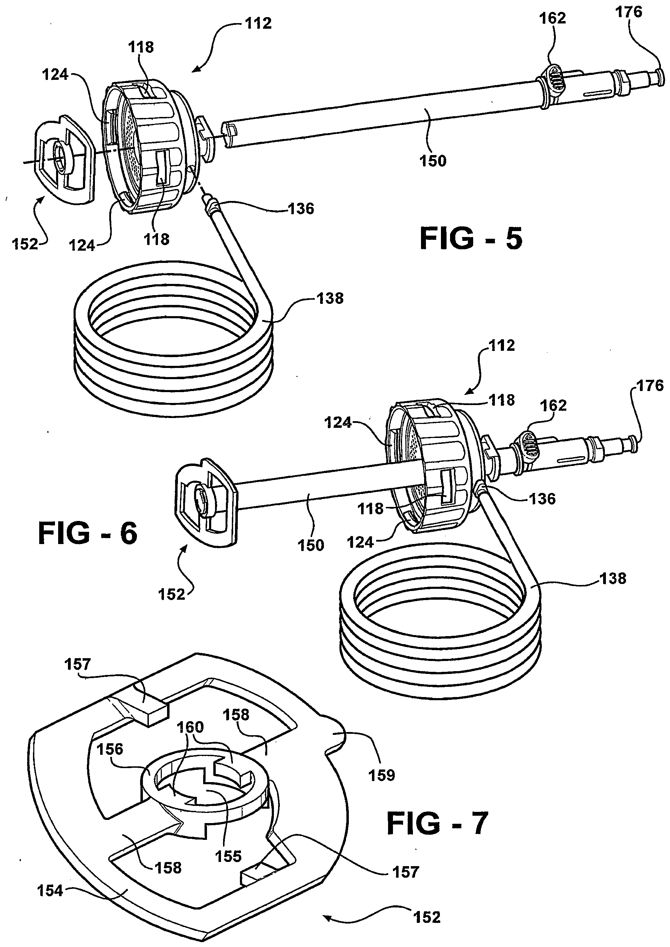

Referring exemplarily to FIG. 4, an o-ring seal 126 and dynamic seal 128 operate together within an orifice 130 in the cap 112 to movably support and seal to the mixing shaft 150. The mixing shaft 150 slides through the orifice 130 and the dynamic seal 128 and is movably supported therein. The dynamic seal 128 allows nearly frictionless rotational, as well as axial movement of the mixing shaft 150 within the mixing chamber 108 to mix the liquid and powder components of the bone cement, while maintaining a snug fit within the orifice 130. A filter 132 and liner 134 are positioned on an interior of the cap 112 to allow a vacuum to be drawn in the mixing chamber 108 by way of a vacuum port 136. The vacuum port 136 is isolated from the mixing chamber 108 by the filter 132 and liner 134 to prevent fouling of a vacuum pump (not shown). Referring to exemplary FIGS. 5-6 which are useful for understanding the invention, a vacuum tube 138 is shown attached to the vacuum port 136 to draw the vacuum in the mixing chamber 108 during mixing.

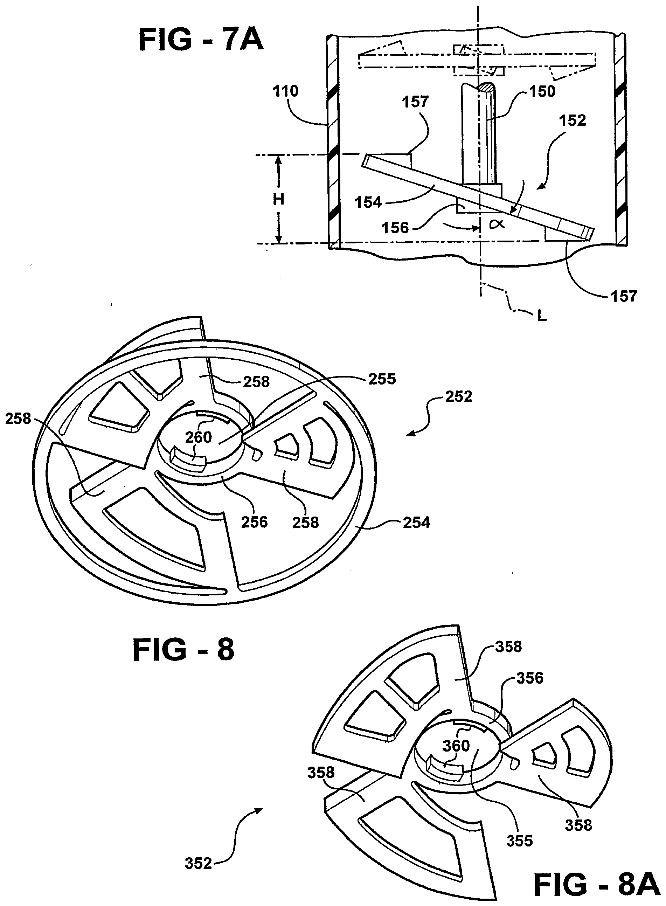

Referring exemplarily to FIG. 7 which is useful for understanding the invention, the preferred blade 152 used to mix the bone cement is shown. The blade 152 is integrally formed from plastic in one piece and has an outer ring 154 connected to a center hub 156 by vanes 158. Ears 160 protrude radially inwardly from the center hub 156 to facilitate a releasable connection to the mixing shaft 150. The releasable connection is described further below. Referring to exemplary FIG. 7A, the outer ring 154 forms an acute angle α with the longitudinal axis L of the cylinder 102 (which is also a rotational mixing axis of the blade 152). The acute angle α is important for efficient mixing of the bone cement. The acute angle α is preferably between twenty and seventy degrees, and more preferably sixty degrees. The blade 152 has an effective height H that is greater than one quarter inch to ensure adequate mixing. Preferably, the effective height H of the blade 15 2is approximately one half inch.

Referring back to exemplary FIG. 7, two radially inwardly protruding fingers 157 are attached to the outer ring 154. One of the fingers 157 protrudes radially inwardly in a first plane and the other finger 157 protrudes radially inwardly in a second plane spaced from and parallel to the first plane. The center hub 156 is positioned between the planes. The fingers 157 are used to scrape proximal and distal regions of the mixing chamber 108 to ensure complete mixing. A protruding node 159 is also attached to the outer ring 154. The node 159 protrudes radially outwardly to control spacing between the blade 152 and an inner periphery of the cylinder wall 110 by scraping along the inner periphery of the cylinder wall 110 in the mixing chamber 108.

FIGS. 8 and 8A only exemplarily illustrate alternative blades 252, 352 that could also be used to mix the bone cement. Each of the blades 152, 252, 352 is designed to flatten at the proximal end 104 of the cylinder 102 adjacent to the cap 112 after the blade 152, 252, 352 is released from the mixing shaft 150 in the mixing chamber 108. This ensures that the maximum possible amount of bone cement can be discharged from the mixing cartridge 100. In the case of the preferred blade 152, the blade 152 is flexible and the outer wall 154 flattens into a plane perpendicular to the longitudinal axis L and occupied by the center hub 156, as illustrated by hidden lines in FIG. 7A. Thus, the effective height H is reduced and the acute angle α becomes close to ninety degrees. This is accomplished by twisting at the vanes 158. Spaces 155, 255, 355 formed in the center hub 156, 256, 356 ensure that once the blade 152, 252, 352 is flattened, the bone cement can pass through the blade 152, 252, 352 when discharged from the mixing cartridge 100. To further facilitate the discharge of the bone cement past the blades 152, 252, 352, each of the center hubs 156, 256, 356 are sized to partially fit within the aperture 130 defined in the cap 112.

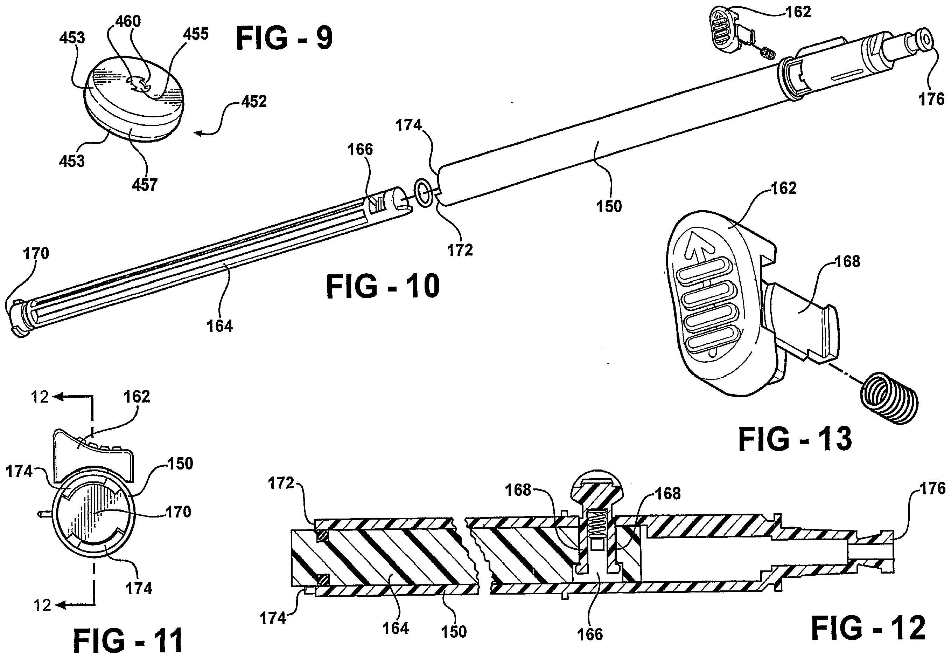

Another alternative blade 452 is shown in exemplary FIG. 9 which is useful for understanding the invention. This blade 452 is a relatively thick disk 452 with chamfered ends 453 forming an acute chamfer angle with a sidewall 457. The chamfer angle is preferably sixty degrees. In the preferred embodiment, the disk is about one half inch thick and about one eighth inch less in diameter than the inner periphery of the cylinder wall 110. In one embodiment, the inner periphery of the cylinder wall 110 is about two and one quarter inches in diameter. As should be appreciated, the slight distance between the side wall 457 of the disk 452 and the inner periphery of the cylinder wall 110 creates a shear force on the bone cement as the disk 452 is rotated and moved axially in the mixing chamber 108. The shear force is the force applied to the bone cement to mix the bone cement. This blade 452 also includes a space 455 formed in a center of the disk 452 and ears 460 for releasably attaching to the mixing shaft 150.

Referring exemplarily to FIGS. 10-13 which are useful for understanding the invention, the mixing shaft 150 has a release latch 162 for releasing the blade 152 from the mixing shaft 150 once mixing of the bone cement is complete. The release latch 162 moves between a holding position and a releasing position. In the holding position, the blade 152 is secured to the mixing shaft 150 to mix the bone cement in the mixing chamber 108. In the releasing position, the blade 152 is released from the mixing shaft 150 to remain in the mixing chamber 108 while the mixing shaft 150 is removed from the cap 112 to make way for a nozzle 204, as will be described further below. The release latch 162 is operatively connected to a latch rod 164, which latches the blade 152 to the mixing shaft 150 in the holding position. The latch rod 164 defines a split cavity 166 for receiving split legs 168 of the release latch 162 in a snap-fit manner. The latch rod 164 is rotatably supported within the mixing shaft 150.

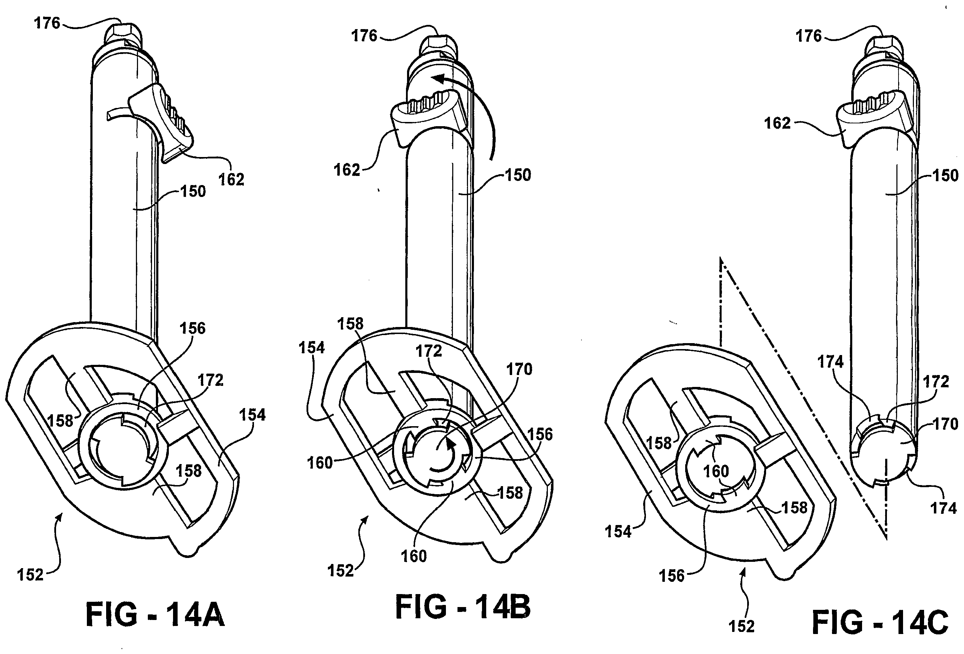

Referring exemplarily to FIGS. 14A-14C which are useful for understanding the invention, the transition of the release latch 162 between the holding position and the releasing position is illustrated. Referring first to FIG. 14C, the exposed end 170 of the latch rod 164 is generally "T" shaped. The corresponding end 172 of the mixing shaft 150 has opposed notches 174 that are adapted to receive the ears 160 on the center hub 156 of the blade152. Initially, the ears 160 are positioned in the notches 174 and the exposed end 170 is positioned over the ears 160 to hold the blade 152 to the mixing shaft 150. See FIG. 14A. To release the blade 152, the release latch 162 is depressed and rotated. Rotating the release latch 162 rotates the latch rod 164 with respect to the mixing shaft 150 thus rotating the exposed end 170 away from the ears 160 to release the blade 152. See FIG. 14B. With the blade 152 released, the mixing shaft 150 is withdrawn from the cap 112 while the blade152 remains in the mixing chamber 108.

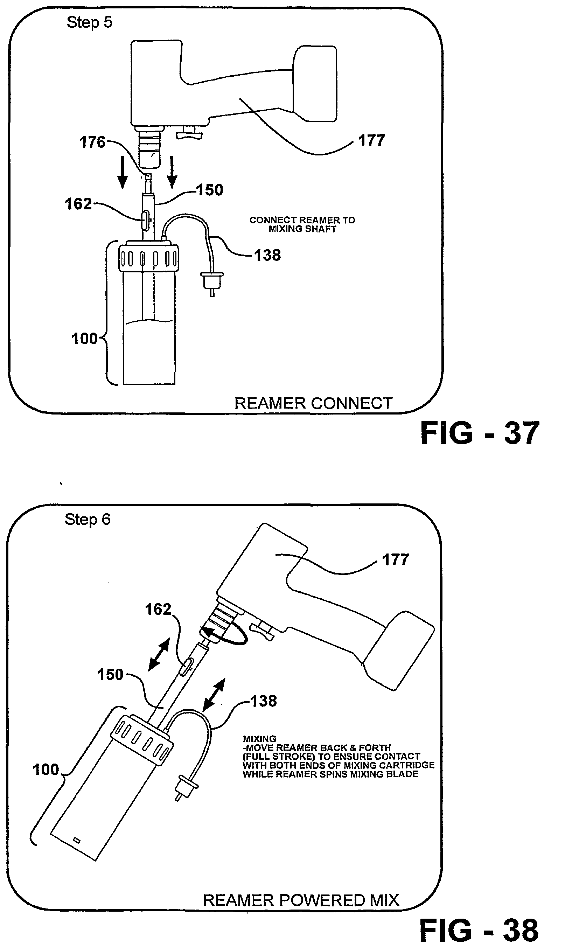

A proximal end 176 of the mixing shaft 150, which represents a portion of the mixing shaft 150 extending outside of the mixing chamber 108 during mixing, is adapted to engage a rotary power tool 177 (see FIG. 37), such as a reamer drill, used to rotate the mixing shaft 150 and blade 152 and mix the bone cement. The proximal end 176 of the mixing shaft 150 is operatively connected to the blade 152 to transfer the rotation of the rotary power tool 177 to the blade 152. When the blade 152 is released from the mixing shaft 150, the operative connection is removed. The operative connection is also removed if the portion of the mixing shaft 150 extending outside of the mixing chamber 108 is severed from the rest of the mixing shaft 150 in the mixing chamber 108, as in alternative embodiments. A manually operated mixing handle (not shown) could engage the mixing shaft 150 at the proximal end 176 to mix the bone cement in other embodiments.

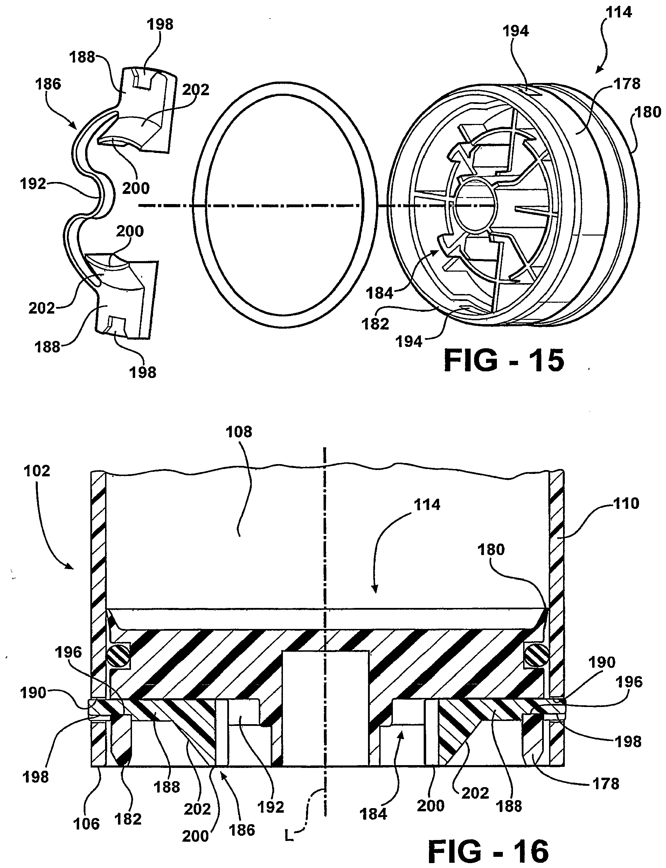

Referring exemplarily to FIGS. 15-16 which are useful for understanding the invention, the piston 114 is positioned within the distal end 106 of the cylinder 102 to further seal the mixing chamber 108. The piston 114 has a skirt 178 extending about the inner periphery of the cylinder wall 110. The piston 114 also includes a proximal end 180 and a distal end 182 defining a cavity 184.

Referring specifically to FIG. 16, the piston 114 is releasably secured in a locked position in the distal end 106 of the cylinder 102 by a locking member 186. The locking member 186 is disposed in the cavity 184 and includes diametrically opposed locking tabs 188 protruding into diametrically opposed slots 190 defined in the cylinder wall 110 to secure the piston 114 to the cylinder 102. It should be appreciated that the slots 190 could be in the form of any suitable female portion, e.g., slot, groove, channel, etc., used for interlocking with a corresponding male portion such as the locking tabs 188. Furthermore, while the embodiment of FIG. 16 illustrates two-way locking, i.e., the piston 114 being locked from moving proximally and distally, the locking member 186 could also be used for one-way locking, i.e., for preventing only proximal movement of the piston 114.

The locking member 186 is integrally formed from plastic and a resilient portion 192 of the locking member 186 biases the locking tabs 188 radially outwardly from the longitudinal axis L into the slots 190. The resilient portion 192 is in the form of a thin resilient ribbon 192 acting like a spring and extending is a winding shape between the locking tabs 188. The locking tabs 188 couple the locking member 186 to the piston 114 by protruding through carrier slots 194 formed in the skirt 178. In the preferred embodiment, a step 196 protrudes into each of the carrier slots 194 to define a guide for sliding engagement within a channel 198 partially defined in each of the locking tabs 188. In the locked position, the carrier slots 194 are axially and radially aligned with the slots 190 formed in the cylinder wall 110.

The piston 114 is locked at the distal end 106 of the cylinder 102 while the liquid and powder components are added and mixed in the mixing cartridge 100. The piston 114 is released from the locked position after mixing of the bone cement is complete. Release buttons 200, integrally formed with the locking tabs 188, are used to release the piston 114 from the locked position. The release buttons 200 are disposed on the locking tabs 188 and protrude distally therefrom. Each of the release buttons 200 includes a cam surface 202 forming an acute angle with the longitudinal axis L. The piston 114 is released from the locked position by squeezing the release buttons 200 radially inwardly against the bias of the resilient portion 192 to withdraw the locking tabs 188 from the slots 190. This action can be performed either manually or mechanically, as will be described further below. After release from the slots 190, the locking tabs 188 remain coupled to the piston 114 in the carrier slots 194.

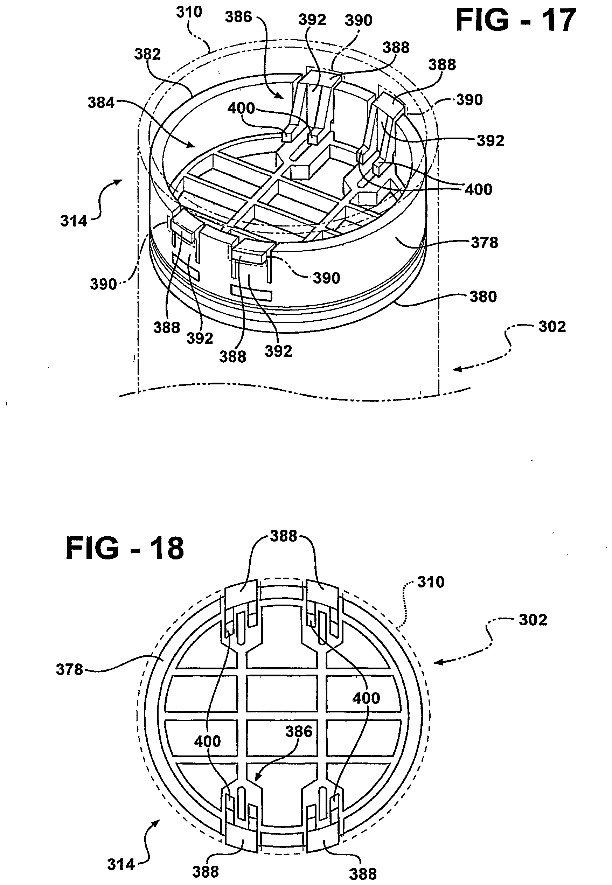

Referring exemplarily to FIGS. 17-18 which are useful for understanding the invention, an alternative locking member 386 is shown. The alternative locking member 386 includes locking tabs 388 that are biased radially outwardly from the longitudinal axis L of the cylinder 302 to engage the slots 390 in the cylinder wall 310. In this embodiment, four slots 390 are defined in the cylinder wall 310 to receive the locking tabs 388. The resilient portion 392 is further defined as a resilient base 392 resiliently supporting each of the locking tabs 388 on the piston 314 with each of the locking tabs 388 being radially biased outwardly from the skirt 378 of the piston 314 to engage the slots 390 in the cylinder wall 310. The release buttons 400 are further defined as fingers 400 extending radially inwardly toward the longitudinal axis L of the cylinder 302 with the fingers 400 being engageable to urge the locking tabs 388 radially inwardly and withdraw the locking tabs 388 from the slots 390 in the cylinder wall 310 to release the piston 314 from the locked position.

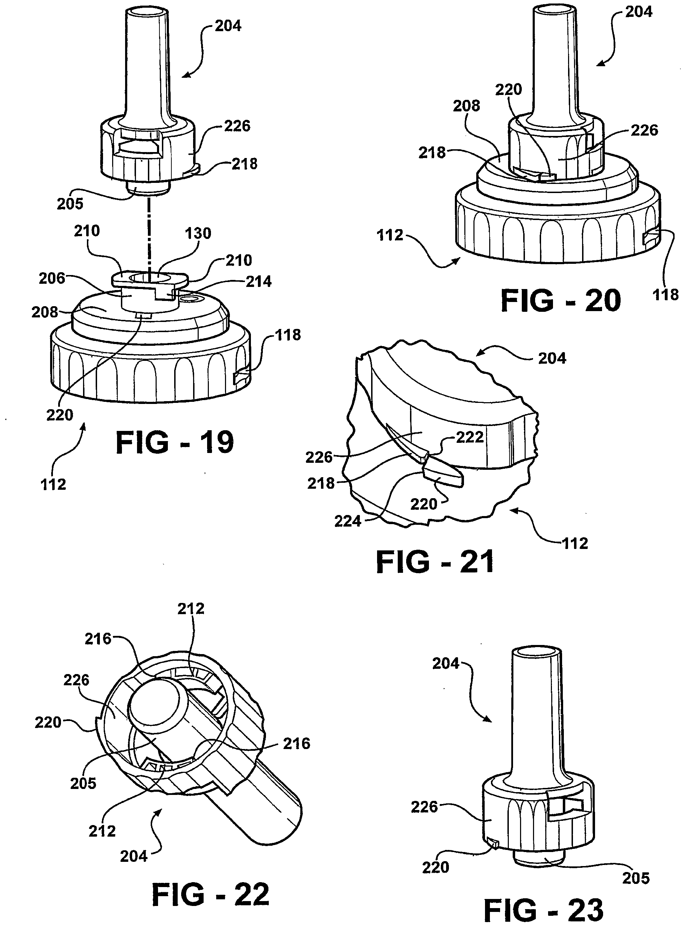

Referring to exemplarily FIGS. 19-23 which are useful for understanding the invention, once the bone cement is mixed, and the mixing shaft 150 is withdrawn from the cap 112, the nozzle 204 is positioned on the cap 112. In the disclosed embodiment, the nozzle 204 is set in place by pushing a hollow shaft 205 of the nozzle 204 down into the orifice 130 of the cap 112 and then twisting the nozzle 204 slightly, about one-quarter turn. The nozzle 204 is attached to the cap 112 to prepare the mixing cartridge 100 for placement into the delivery gun 500.

The cap 112 has a nipple 206 protruding from an outer surface 208t hereof. The nipple 206 has tabs 210, which engage detent members 212 in the nozzle 204. After the nozzle 204 is fully rotated into position, the tabs 210 fully engage the detent members 212 while being positioned proximal to the detent members 212 to secure the nozzle 204 in place. A stop 214 on the cap 112, best shown exemplarily in FIG. 19, prevents the nozzle 204 from rotating freely in the clockwise direction after the tabs 210 have engaged the detent members 212. The stop 214 extends downwardly from one of the tabs 210 to abut a side surface 216 of one of the detent members 212 to prevent further clockwise rotation.

The nozzle 204 and cap 112 have first 218 and second 220 locking protrusions. The first locking protrusion 218 acts as a detent and slides over the second locking protrusion 220 to a locked position as illustrated as an example in FIG. 21. In this position, rear flat surfaces 222, 224 of the locking protrusions 218, 220, abut one another to prevent the nozzle 204 from being turned in the opposite direction, thereby preventing removal of the nozzle 204 from the cap 112.The nozzle 204 can be removed by deflecting an outer skirt 226 of the nozzle 204 and rotating the nozzle 204 counterclockwise thereby disengaging the locking protrusions 218, 220. Both the nozzle 204 and cap 112 are formed from plastic, which facilitates the detent-like locking and unlocking of the nozzle 204 to the cap 112.

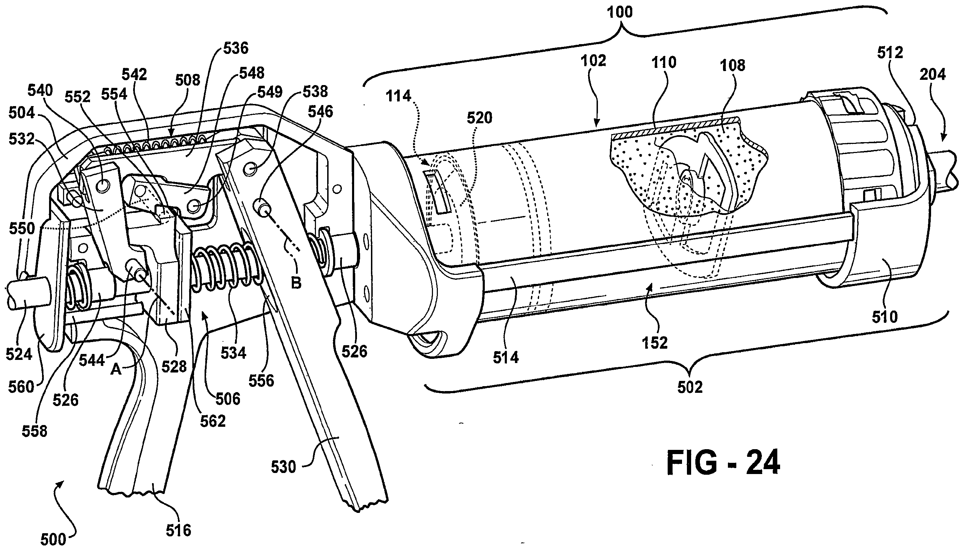

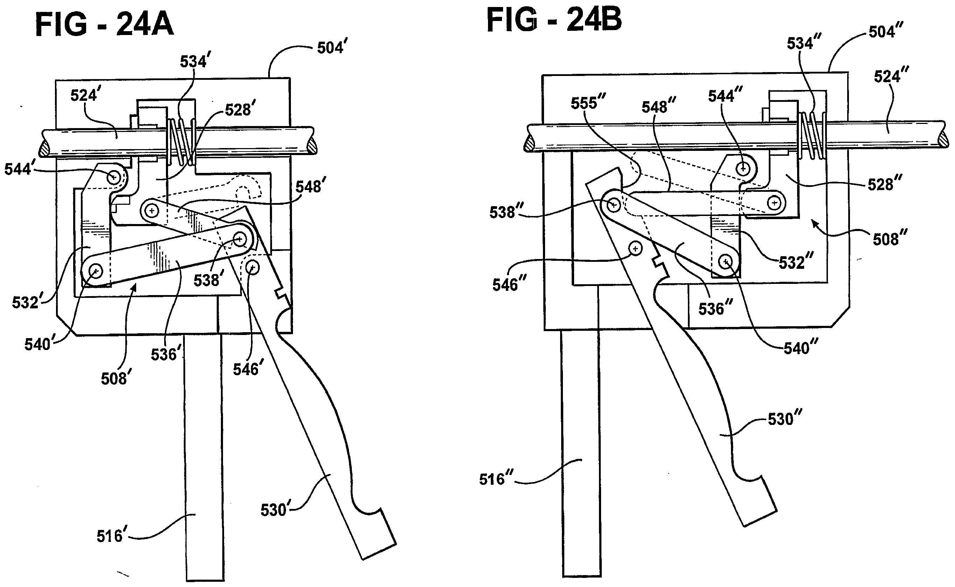

With the nozzle **204** in place, the mixing cartridge **100** is ready to be placed within the delivery gun **500.** Referring to FIG. 24, the delivery gun **500** of the present invention includes a cradle **502** for supporting the mixing cartridge **100** and a casing **504** fixed to the cradle **502** for supporting a drive mechanism **506,** a linkage system **508,** and corresponding components. The cradle **502** includes an endplate **510,** which has an opening **512** for receipt of the nozzle **204.** The endplate **510** holds the mixing cartridge **100** in position in the cradle **502.** In the preferred embodiment, the casing **504** and the endplate **510** are connected by two connecting bars **514** (one on each side of the mixing cartridge **100**) to reduce the weight of the delivery gun **500.** A handle **516** is integrally formed with the casing **504** to maneuver the delivery gun **500** during use.

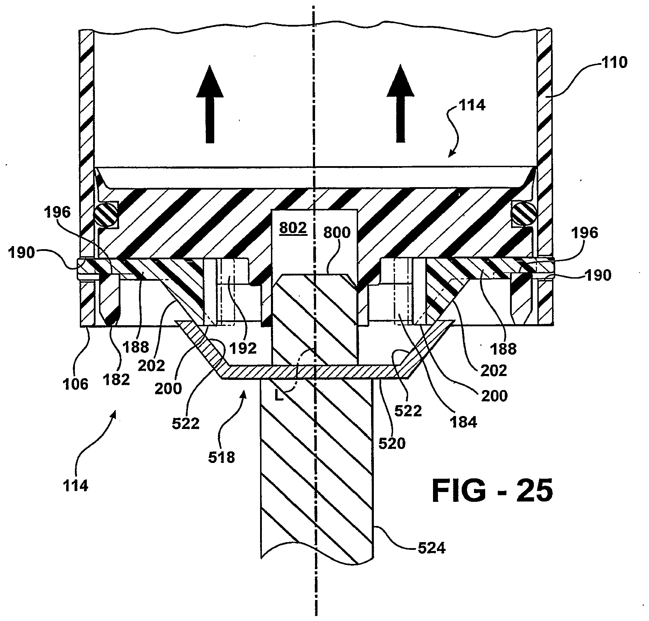

To dispense the bone cement from the mixing cartridge **100,** the piston **114** must first be released from the locked position. Referring to FIG. 25, this is accomplished using a release mechanism **518** integrated into the delivery gun **500.** Once the mixing cartridge **100** is in place in the cradle **502,** a ram disk **520** protrudes into the cavity **184** in the distal end **182** of the piston **114.** The release mechanism **518** is integrated into the ram disk **520.** The release mechanism **518** includes a bearing surface **522** forming an acute angle with the longitudinal axis **L** for catching the release buttons **200** to cam the release buttons **200** radially inwardly. More specifically, the cam surfaces **202** of the release buttons **200** slide along the bearing surface **522,** while being cammed radially inwardly. This action pulls the locking tabs **188** radially inwardly to withdraw the locking tabs **188** from the slots **190** in the cylinder wall **110** and release the piston **114** from the locked position (when the alternative piston **314** is used, the ram disk has a flat bearing surface that axially presses the fingers **400** proximally to bend each resilient base **392** inwardly and urge the locking tabs **388** radially inward). A centering pin **800** can be used to center the ram disk **520** in a centering cavity **802** of the piston **114** to facilitate the release of the piston **114** from the locked position.

Referring back to FIG. 24, once the piston **114** is released, the piston **114** can be driven through the mixing chamber **108** by the drive mechanism **506** to force the bone cement from the nozzle **204.** The drive mechanism **506** includes a drive rod **524** movably supported by bushings **526** in the casing **504.** The ram disk **520** is fixed to the drive rod **524.** The drive mechanism **506** further includes a first gripper plate **528** responsive to movement of the linkage system **508** upon actuation of a trigger **530.** The first gripper plate **528** defines an aperture surrounding the drive rod **524.** The first gripper plate **528** frictionally engages the drive rod **524** to advance the drive rod **524.** The first gripper plate **528** is urged forward while in frictional contact with the drive rod **524** by the linkage system **508** when the trigger **530** is actuated. The first gripper plate **528** thereby advances the drive rod **524** and ram disk **520** relative to the casing **504** to drive the piston **114** and force the bone cement from the mixing cartridge **100.** The trigger **530** is pivotally supported by the casing **504** and operatively connected to the drive mechanism **506** to advance the drive mechanism **506** upon actuation of the trigger **530.**

The linkage system **508** includes a first link **532,** which is pivotally mounted to the casing **504** about a pivot axis **A** adjacent to the first gripper plate **528.** The first link **532** is adapted to engage the first gripper plate **528** when the first link **532** pivots about the pivot axis **A.** A second link **536** pivotally interconnects the trigger **530** to the first link **532** via support pins **538, 540.** The links **532, 536** and trigger **530** are interconnected to move in unison upon rotation of the trigger **530** about a second pivot axis **B**. When the trigger **530** is pulled, the second link **536** rotates the first link **532** about the pivot axis **A,** which engages the first gripper plate **528** and urges the first gripper plate **528** forward while the first gripper plate **528** is in frictional engagement with the drive rod **524** thereby advancing the drive rod **524.** A return spring **542** returns the links **532, 536** and the trigger **530** to an initial position upon release of the trigger **530.** At the same time, a first spring **534** momentarily disengages the first gripper plate **528** from the drive rod **524** to slide the first gripper plate **528** back to an initial position to await the next pull of the trigger **530.** The casing **504** pivotally supports the first link **532** and the trigger **530** about the pivot axes **A** and **B** via support pins **544, 546.**

A speed-changing link **548** is pivotally connected to the second link **536** about a support pin **549.** The speed-changing link **548** selectively pivots into and out from engagement with the first gripper plate **528** by way of a switch **550.** The speed-changing link **548** pivots between a high-speed position and a low-speed position about the support pin **549** (the low-speed position is shown in FIG. 24). The high-speed position corresponds to faster advancement of the drive rod **524** at a lower force. This allows the user to quickly advance the drive rod **524** to drive the piston **114** and dispense high volumes of bone cement at low pressure. The low-speed position corresponds to slower advancement of the drive rod **524** at a higher force, which exerts more force on the piston **114** to pressurize the bone cement.

The first gripper plate **528** and the speed-changing link **548** have complementary first and second coupling devices **552, 554** used to couple the first gripper plate **528** with the speed-changing link **548** in the high-speed position. More specifically, in the embodiment of FIG. 24, the first gripper plate **528** has a shoulder **552** that is received within a channel **554** on the speed-changing link **548.** The speed-changing link **548** engages the shoulder **552** in the high-speed position. In the high-speed position, a user's gripping force is transmitted through the trigger **530** to the second link **536** and the speed-changing link **548** to engage the first gripper plate **528** and advance the drive rod **524.** The speed-changing link **548** is isolated from the first gripper plate **528** in the low-speed position. The low-speed position corresponds to the speed-changing link **548** being switched or disconnected from the shoulder **552.** In the low-speed position, the user's gripping force is transmitted through the trigger **530** to both the first **532** and second **536** links to engage the first gripper plate **528** and advance the drive rod **524.** This results in slower advancement of the drive rod **524,** but at a much higher mechanical advantage than the high-speed position. As a result, the user can better pressurize the bone cement during injection.

The pivot axes **A** and **B** and the links **532, 536, 548** are positioned above the drive rod **524,** while the trigger **530** extends below the drive rod **524.** A channel **556** defined in the trigger **530** facilitates this configuration. There are several advantages to this configuration. Moving the second pivot axis **B** away from a user's hand results in better usage of the stronger index and ring fingers by allowing those fingers more travel distance as the trigger **530** is actuated. This configuration also allows the handle **516** to be closer to the drive rod **524,** which is believed to reduce wrist strain when the user pushes the delivery gun **500** forward during cement pressurization. Another benefit is that it allows for a more streamlined casing design and better weight distribution.

In one embodiment, shown in FIG. 24, a secondary gripper plate **562** is mounted about the drive rod **524** adjacent to the first gripper plate **528.** The addition of one or more secondary gripper plates **562** to the first gripper plate **528** adds strength to the delivery gun **500** while still permitting proper operation. By using two or more gripper plates **528, 562,** increased frictional contact with the drive rod **524** is obtained without adversely affecting performance.

A release pin **558** disengages the gripper plates **528, 562** to allow a user to freely move the drive rod **524** by hand. The release pin **558** is connected to a retainer plate **560** and is adapted to engage the first gripper plate **528.** When the retainer plate **560** is pushed by the user, the release pin **558** engages the first gripper plate **528** which forces the first gripper plate **528** to tilt back against the bias of the first spring **534** thus releasing the drive rod **524**. Any secondary gripper plates **562** follow. As should be appreciated, pushing the retainer plate **560** also pivots the retainer plate **560** releasing its engagement with the drive rod **524.** With both the retainer plate **560** and the gripper plates **528, 562** released, the drive rod **524** is free to move. This allows the user to manually move the drive rod **524** with respect to the casing **504.**

The delivery gun **500** is unique among bone cement guns with a friction-plate mechanism in the way that it handles wear and deformation of the gripper plates **528, 562.** In the disclosed embodiments, the gripper plates **528, 562** are tilted by the first spring **534** into frictional contact with the drive rod **524.** Regardless of the amount of wear or deformation of the gripper plates **528, 562** or the drive rod **524,** the gripper plates **528, 562** require no further tilting to engage the drive rod **524** upon actuation of the trigger **530.** Thus, advancement of the drive rod **524** is produced over the entire actuation of the trigger **530** and efficiency is maintained throughout the life of the delivery gun **500.**

Referring to FIGS. 24A and 24B, alternatives of the linkage system **508'** and **508"** are shown. These alternatives are represented with similar numerals to the embodiment of FIG. 24 to indicate like parts. FIG. 24A illustrates a configuration of the linkage system **508'** in which the linkage system **508'** lies beneath the drive rod **524'**. Furthermore, the speed-changing link **548'** in this embodiment is pivotally connected to the first gripper plate **528'** and includes a hook-shaped end to engage the support pin **538'** in the high-speed position and disengage the support pin **538'** in the low-speed position. FIG. 24B illustrates a configuration of the linkage system **508"** in which the first gripper plate **528"** is pushed by the linkage system **508",** as opposed to being pulled by the linkage system **508** and **508'** in FIGS. 24 and 24A. Here, the speed-changing link **548"** is pivotally connected to the first gripper plate **528"** to pivot into engagement with a notch **555"** defined in the trigger **530"** in the high-speed position and out from engagement with the notch **555"** in the low-speed position. These alternatives of the linkage system **508'** and **508"** illustrate the flexibility of design, e.g., the selection of mechanical advantage, provided by the linkage system of the present invention.

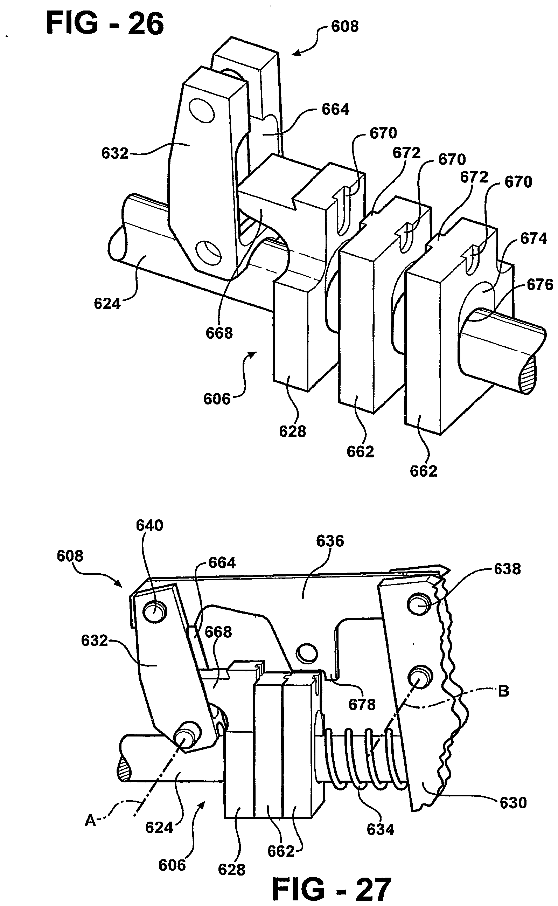

Referring to FIGS. 26-27, an alternative embodiment of the drive mechanism **606** and linkage system **608** is shown (only a portion of the drive mechanism **606** and linkage system **608** is shown for illustrative purposes). In this embodiment, the linkage system **608** comprises the same components as previously described with an improved first link **632** and gripper plates **628, 662.** In this embodiment, a plurality of secondary gripper plates **662** are aligned along the drive rod **624** next to the first gripper plate **628.** The first link **632** defines a female recess **664** and the first gripper plate **628** includes a male member **668** for mating engagement with the female recess **664.** The secondary gripper plates **662** are aligned relative to the first gripper plate **628** via mating notches **670** and pegs **672** formed therein. The notches **670** and pegs **672** assume the same shape to mate with one another and maintain alignment. This arrangement minimizes alignment changes that may cause slipping or uneven wear. The arrangement also reduces contact between the gripper plates **628, 662** and an interior wall of the casing **504.** The gripper plates **628, 662** are shown spaced in FIG. 26 for illustration only. In practice, the gripper plates **628, 662** abut one another, as shown in FIG. 27.

In this embodiment, each of the gripper plates **628, 662** also defines a pair of semi-spherical grooves **674.** In FIG. 26, only the first of the pair of grooves **674** are shown in each of the gripper plates **628, 662.** The other of the pair of grooves **674** is located in a rear surface of each of the gripper plates **628, 662,** cater-cornered from the first of the pair of grooves **674.** These grooves **674** increase the frictional contact with the drive rod **624.** When the gripper plates **628, 662** are urged forward while in frictional engagement with the drive rod **624** by the first link **632,** a substantial portion of a rim **676** defined by each of the grooves **674** frictionally contacts the drive rod **624.**

Referring to FIG. 27, autoclave sterilization of the delivery gun **500** can create a tendency for the gripper plates **628, 662** to adhere to the drive rod **624** beyond their initial positions when the trigger **630** is released. In this situation the first spring **634** cannot produce enough force to disengage the gripper plates **628, 662** from the drive rod **624,** and the gripper plates **628, 662** do not return to their initial positions. FIG. 27 shows a way to prevent this condition. A striker **678,** in the form of a downwardly protruding portion of the second link **636,** closely follows one of the gripper plates **628, 662** during actuation of the trigger **630.** In the event that any of the gripper plates **628, 662** do not properly disengage the drive rod **624** upon release of the trigger **630,** the striker **678** will contact the notch **670** in the closest gripper plate **628, 662** and dislodge the gripper plate **628, 662** from the drive rod **624.** The first spring **634** can then properly return the gripper plates **628, 662** to their initial positions.

A coating has been added to an exterior of each of the gripper plates **528, 562, 628, 662** in FIGS. 24 and 26-27. The coating increases lubricity and corrosion resistance. This facilitates sliding between the gripper plates **528, 562, 628, 662** as they engage the drive rod **524, 624.** The coating also reduces corrosion due to autoclave sterilization that may cause the gripper plates **528, 562, 628, 662** to adhere to one another and prevent proper engagement with the drive rod **524, 624.** The coating used may be Electroless-Nickel with polytetrafluoroethylene (PTFE) or other like coatings possessing the same or similar properties.

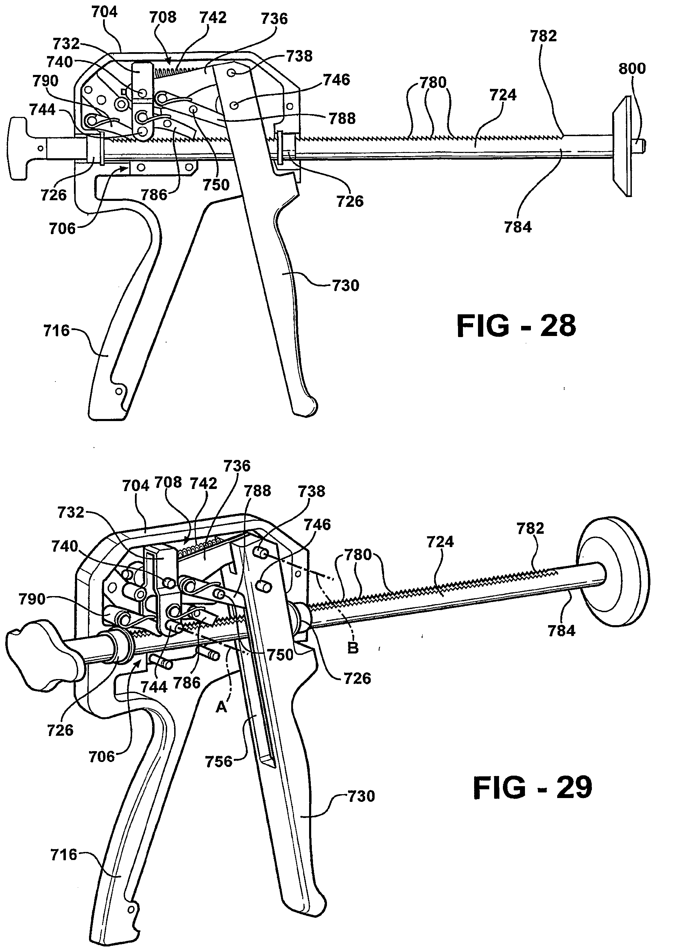

Referring to FIGS. 28-31, another alternative embodiment of the drive mechanism **706** and linkage system **708** is shown. This embodiment also provides selective high-speed and low-speed advancement of the drive rod **724.** This alternative drive mechanism **706** eliminates the gripper plate by providing teeth **780** on the drive rod **724.** A cross-section of the drive rod **724** shows the teeth **780** on a flat upper surface **782,** while a lower surface **784** is smooth and round. The first link **732,** which in previous embodiments urged the first gripper plate **528, 628** forward with the drive rod **524, 624,** now pivotally supports a first pawl member **786.** The first pawl member **786** is spring-biased into engagement with the teeth **780.**

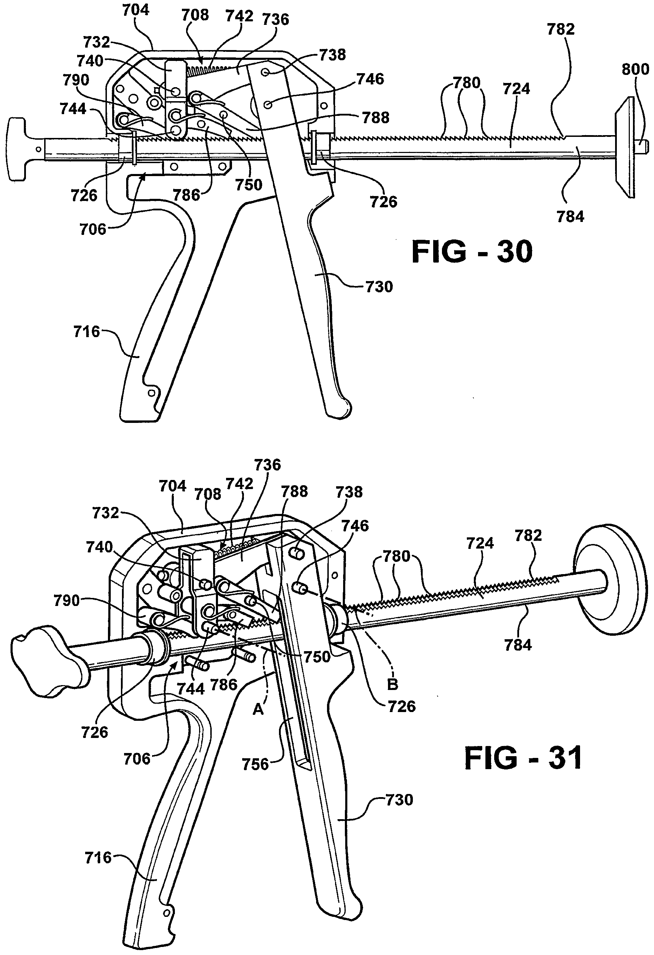

A second pawl member **788** is pivotally supported by the second link **736.** The second pawl member **788** is pivotable between a high-speed position in which the second pawl member **788** is spring-biased into engagement with the teeth **780** to advance the drive rod **724,** and a low-speed position in which the second pawl member **788** is disengaged and isolated from the teeth **780.** In the low-speed position, the first pawl member **786** advances the drive rod **724.** The low-speed position is illustrated in FIGS. 28-29. In the high-speed position, with the second pawl member **788** engaging the teeth **780,** the first pawl member **786** remains in engagement with the teeth **780,** but only ratchets along the teeth **780** as the second pawl member **788** advances the drive rod **724.** The high-speed position is illustrated in FIGS. 30-31. The principle of increasing mechanical advantage in the low-speed position relative to the high-speed position also applies in this embodiment.

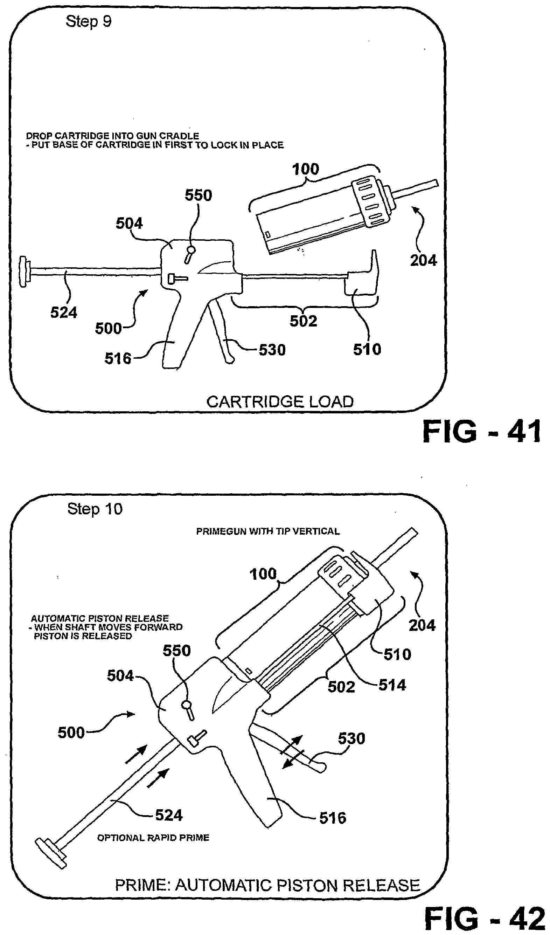

The switch **750** is used to pivot the second pawl member **788** out from engagement with the teeth **780** of the drive rod **724** in the low-speed position (see FIGS. 28-29) and into engagement with the teeth **780** in the high-speed position (see FIGS. 30-31). A switch similar to that shown in United States Patent No. 5,431,654 to Nic, herein incorporated by reference, can be used for this purpose. The switch **750** extends through the casing **704** and terminates in a button that is manipulated by a user to move the second pawl member **788** between the high-speed and low-speed positions (see briefly FIGS. 41-42). This also applies to the switch **550** used to move the speed-changing link **548** in previous embodiments.

In this embodiment, the retainer plate **560** can be removed. In its place, a spring-biased non-return pawl member **790** retains the drive rod **724** in position upon advancement. The drive rod **724** can be freely moved in the casing **704** by rotating the drive rod **724** one hundred and eighty degrees such that the pawl members **786, 788, 790** are out of engagement with the teeth **780.** Upon such rotation, the pawl members **786, 788, 790** ride on the smooth lower surface **784** of the drive rod **724** allowing the user to freely pull the drive rod **724** relative to the casing **704.** This is generally disclosed in the '654 patent to Nic.

Each of the pawl members **786, 788, 790** are pivotally supported by pins. Springs, such as those shown in the '654 patent to Nic, bias the pawl members into engagement with the teeth **780** on the drive rod **724** (except when the switch **750** acts against the bias of the spring in the low-speed position to disengage the second pawl member **788** from the teeth **780**).

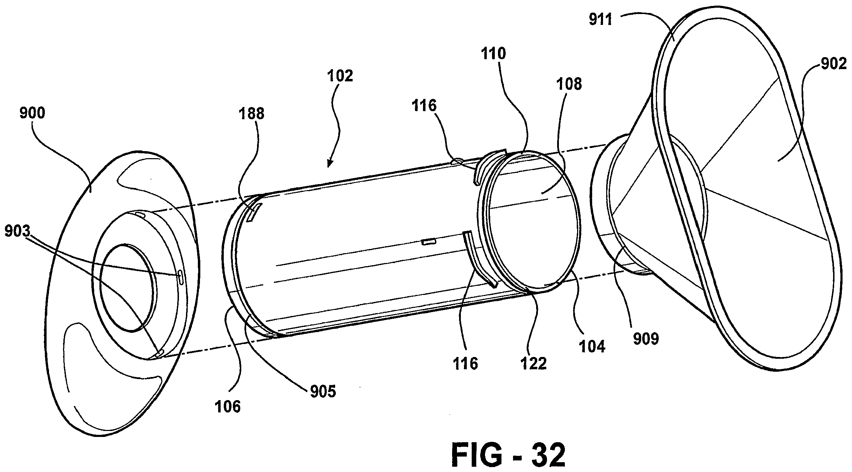

Mixing and delivery of the bone cement will now be described with reference to FIGS. 32-42. Referring first to FIG. 32, a bone cement loading system is shown. The bone cement loading system comprises a base **900** supporting the cylinder **102** while loading the liquid and powder components of the bone cement into the mixing chamber **108.** The base includes a cavity for receiving the distal end **106** of the cylinder **102.** Detents **903** are formed in the cavity. A groove **905** is defined in an outer surface of the cylinder **102** to receive the detents **903** and facilitate a snug fit between the base **900** and the cylinder **102.** It should be appreciated that the detents **903** could be formed on the cylinder **102** with the groove **905** defined in the base **900.** The distal end **106** of the cylinder **102** may also be press fit into the base **900.** The base **900** is oblong and oval in shape to fully support the cylinder **102** on a work surface, while the cavity is circular in shape to fit the circular shaped cylinder **102.** A funnel **902** couples to the cylinder **102** to channel the powder into the cylinder **102** during loading. The funnel **902** includes a proximal end **911** having an oblong oval-shaped periphery to facilitate the loading of the powder into the mixing chamber **108** and a distal end **909** having a circular periphery to snugly fit inside the proximal end **104** of the cylinder **102.**

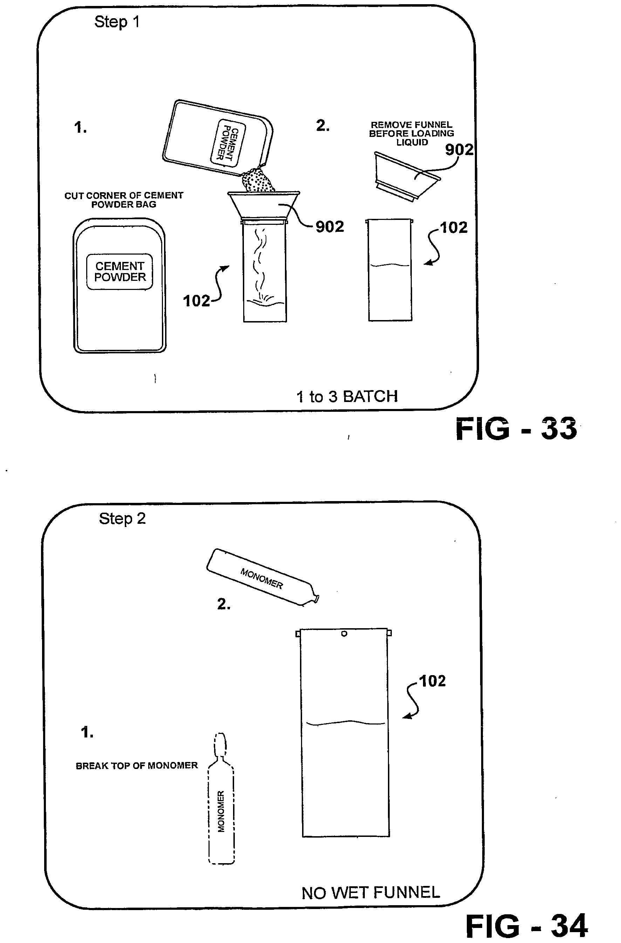

FIGS 33-42 illustrate ten steps for preparing and injecting the bone cement. The mixing cartridge **100**, delivery gun **500,** and other components are generically shown in each step for illustrative purposes only.

In STEP 1, shown in FIG. 33, the funnel **902** is coupled to the cylinder 102 and the powder is poured into the mixing chamber **108.**

In STEP 2, shown in FIG. 34, after the powder is poured into the mixing chamber **108,** the funnel **902** is removed, and the liquid component, e.g., liquid monomer, of the bone cement is added. In this manner, the present invention avoids wetting of the funnel **902** and the associated clean-up.

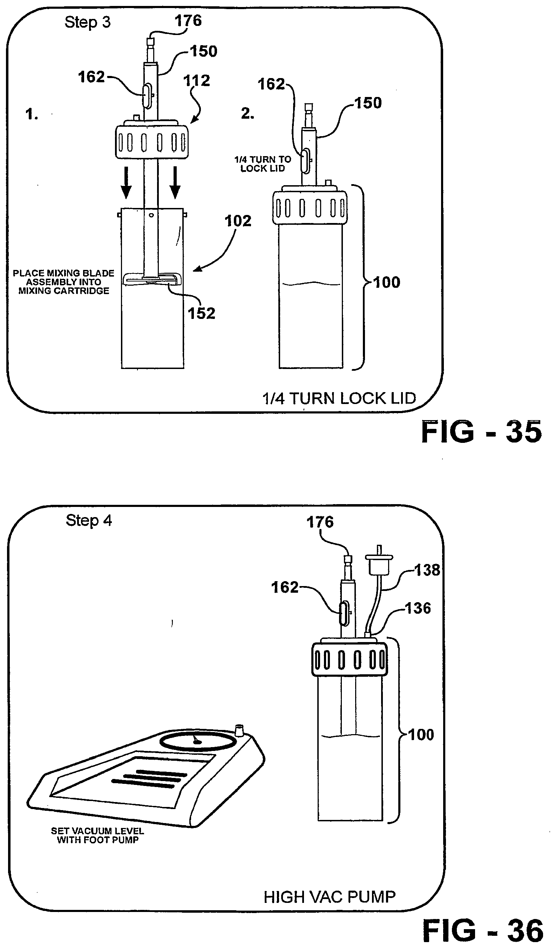

In STEP 3, shown in FIG. 35, the cap **112** with the mixing shaft **150** and blade **152** supported therein is attached to the cylinder **102.**

In STEP 4, shown in FIG. 36, the vacuum line **138** is attached to the vacuum port **136** and a vacuum is drawn in the mixing chamber **108** with the liquid and powder components therein.

In STEP 5, shown in FIG. 37, with the vacuum drawn, the power tool (reamer) is then connected to the mixing shaft **150.**

In STEP 6, shown in FIG. 38, with the vacuum still drawn, the mixing shaft **150** is moved axially with respect to the mixing cartridge **100** and rotated by the power tool. The blade **152** (not shown in FIG. 38) is moved axially the entire extent of the mixing cartridge **100** while rotating to ensure that the liquid and powder components are fully mixed.

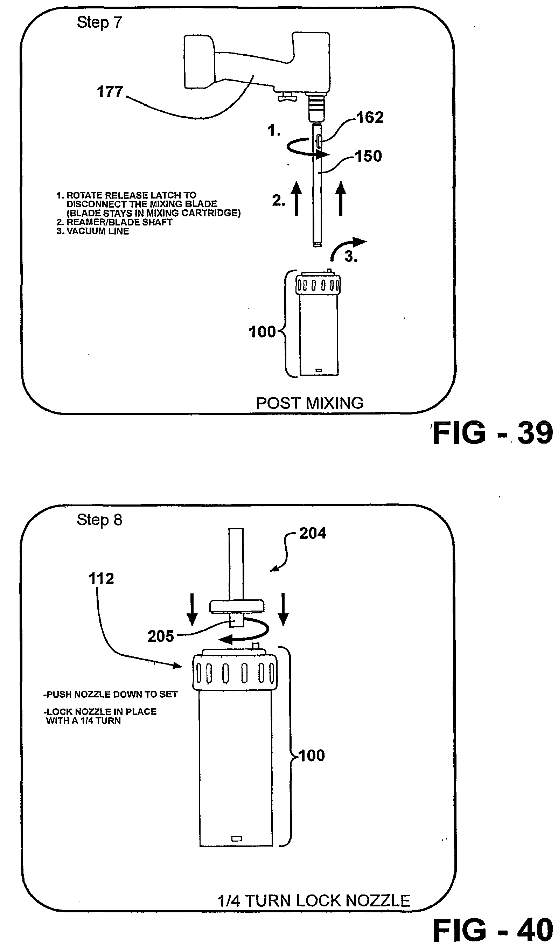

In STEP 7, shown in FIG. 39, once mixed, the release latch **162** is moved to release the blade **152** (not shown in FIG. 39). The blade **152** remains in the mixing chamber **108** once released. The mixing shaft **150** is then removed from the mixing cartridge **100.** Mixing is now complete.

In STEP 8, shown in FIG. 40, the nozzle **204** is pushed down on the cap 112 and rotated into place.

In STEP 9, shown in FIG. 41, the mixing cartridge **100** is positioned in the cradle **502.**

In STEP, shown in FIG. 42, the piston **114** is released from the distal end **106** of the cylinder **102** and the delivery gun **500** is primed and ready to discharge the bone cement from the mixing cartridge **100.**

It will be appreciated that the above description relates to the disclosed embodiments by way of example only. Many apparent variations of the disclosed invention will be known to those of skill in this area and are considered to be within the scope of this invention and are considered to be within the scope of the following claims. Obviously, many modifications and variations of the present invention are possible in light of the above teachings.

## Claims

1. A gun (500) for dispensing bone cement from a cartridge (100), said gun including:
a casing (504, 504', 504", 704);
a cradle (502) attached to the casing, the cradle shaped to support a cartridge (100) containing bone cement;
a drive rod (524, 524', 524", 624, 724) mounted to the casing (504, 504', 504", 704), the drive rod having a forward end shaped to act against a piston (114) integral with the cartridge (100) and the drive rod being moveably mounted to the casing so that the forward end of the drive rod can abut and push the cartridge piston away from the casing; and
a linkage assembly (508, 508' 508", 608, 708), the linkage assembly including:
a trigger (530, 530', 530", 630, 730) pivotally mounted to the casing; and a drive rod advancement member (528, 528', 528", 628, 662, 786) disposed in the casing, the drive rod advancement member being connected to the trigger to be actuated by the trigger and adapted to, upon actuation of the trigger, engage the drive rod (524, 524', 524", 624, 724) so as to advance the drive rod against the piston (114) integral with the cartridge (100) held by the cradle (502);
wherein the linkage assembly (508, 508' 508", 608, 708) further includes:
a first link (532, 532', 532", 632, 732) separate from said trigger, said first link being pivotally mounted to said casing and adapted to engage the drive rod advancement member (528, 528', 528", 628, 786); and
a second link (536, 536', 536", 636, 746) separate from the trigger and said first link, said second link adapted to interconnect said trigger and said first link, wherein said second link moves upon the pivoting of said trigger so that the movement of said second link results in movement of the first link that actuates the drive rod advancement member;
**characterized in that** the linkage assembly (508, 508' 508", 608, 708) further includes:
a third link (548, 548', 548", 788) separate from the trigger, said first link, and said second link, said third link adapted to link the trigger to the drive rod (524, 524', 524", 624, 724) so that the movement of the trigger is transferred through said third link to the drive rod so that said drive rod advances at a higher speed than when said first link actuates said drive rod advancement member, said third link having: an engaged position in which said third link connects the trigger to said drive rod so that the pivoting of said trigger results in the advancement of said drive rod by said third link; and a disengaged position in which said third link does not connect the trigger to said drive rod so that the pivoting of said trigger does not result in said third link advancing said drive rod so that the trigger actuates said first link via said second link resulting in said drive rod advancement member advancing said drive rod; and
a control member (550, 750) adapted to move said third link between the engaged and disengaged positions.

2. The bone cement dispensing gun (500) of Claim 1, wherein:
the drive rod (724) is formed with teeth (780); and
the drive rod advancement member is a first pawl (786) shaped to engage said drive rod teeth when moving said drive rod in a forward direction.

3. The bone cement dispensing gun (500) of Claim 2, wherein said third link is a second pawl (788) that engages said drive rod teeth (780) so that the displacement of said second pawl by the trigger (730) causes said second pawl to move said drive rod in the forward direction.

4. The bone cement dispensing gun (500) of Claims 2 or 3, wherein:
the drive rod (724) is formed so that said teeth (780) extend longitudinally over a first surface of said drive rod and said drive rod is formed with a second surface that is arcuately spaced from the first surface and that is smooth; and
the drive rod is mounted to rotate within said casing (704) so that either said teeth of the drive rod first surface or the second surface can be placed into contact with said first pawl (786).

5. The bone cement dispensing gun (500) of any one of Claims 1, 2 or 4, wherein said linkage assembly third link (548, 548', 548") is positioned to, when in the engaged position, engage the drive rod advancement member (528, 528', 528", 628) so that the displacement of said third link by the trigger (530, 530', 530", 630) results in said third link actuating said drive rod advancement member so that said drive rod advancement member advances said drive rod.

6. The bone cement dispensing gun (500) of Claim 1, wherein said third link (788) is positioned to, when in the engaged position, engage the drive rod (724) so that said third link functions as a component that, when said third link is actuated by the trigger (730), moves said drive rod in the forward direction.

7. The bone cement dispensing gun (500) of any one of Claims 1 to 6, wherein:
the trigger (530, 730) is mounted to the casing (504, 704) so that the trigger extends away from a first side of the drive rod (524, 724) and out of said casing; and
said first link (536, 788), said second link (532,732) and said third link (548, 788) of the linkage assembly (508, 730) are disposed in said casing (504, 704) so as to be located adjacent a second side of said drive rod that is opposite the first side of said drive rod.

8. The bone cement dispensing gun (500) of any one of Claims 1 to 7, wherein the cradle (502) includes:
at least one bar (514) that extends forward from a front end of said casing (504); and
an endplate (510) mounted to said at least one bar so as to be spaced forward from said casing, said end plate shaped to receive an end of the cartridge (100).

9. The bone cement dispensing gun of any one of Claims 1 to 8, wherein:
the trigger (530, 530' 730) is located adjacent a front end of the casing (504, 704); and
the first link (536, 536', 736) is pivotally mounted to the trigger to extend rearwardly away from the front end of said casing.

10. The bone cement dispensing gun (500) of any one of Claims 1, 5, 6, 7, 8 or 9, wherein the drive rod advancement member is a gripping member (528, 528', 628, 662) that frictionally engages the drive rod (524, 524, 524", 624) to advance the drive rod.

11. The bone cement dispensing gun (500) of Claim 10, wherein said gripping member (528, 528', 528", 628) is formed with an aperture through which said drive rod (524, 524', 524", 624) extends.

12. The bone cement dispensing gun (500) of Claims 10 or 11, wherein:
said gripping member (528, 528', 528", 628) is capable of both forward and rearward movement; and
a spring (534, 534' 534", 634) extends between said casing and said gripping member so as to move said gripping member out of engagement with said drive rod when said gripping member undergoes rearward movement.

13. The bone cement dispensing gun (500) of any one of Claims 10, 11 or 12, further including a release pin (558) that is moveably mounted to said casing (504), said release pin having:
a disengaged position in which said release pin is spaced from said gripping member (528); and
an engaged position in which said release pin engages and displaces said gripping member (528) so that said gripping member moves out of engagement with said drive rod.

14. The bone cement dispensing gun (500) of any one of Claims 10, 11, 12 or 13, wherein the drive rod advancement member includes a plurality of gripping plates (528 and 562, 628 and 662) that bear against the drive rod (524, 624).

15. The bone cement dispensing gun (500) of any one of Claims 1 to 14, wherein said third link (548, 788) is pivotally mounted to said second link (536, 736) so that the displacement of said second link by said trigger (530, 730) results in the displacement of said third link by said second link.

16. The bone cement dispensing gun (500) of any one of Claims 1 to 14, wherein said third link (548', 548") is pivotally connected to the drive rod advancement member (528', 528").

17. The bone cement dispensing gun (500) of Claim 16, wherein the trigger (530'), the drive rod advancement member (528') and said third link (548') are collectively mounted to the casing (504) so that, when said third link is in the engaged position, said third link extends forward from said drive rod advancement member.

18. The bone cement dispensing gun (500) of Claim 16, wherein the trigger (530"), the drive rod advancement member (528") and said third link (548") are collectively mounted to the casing (504) so that, when said third link is in the engaged position, said third link extends rearward from said drive rod advancement member.

## Patentansprüche

1. Pistole (500) zum Austragen von Knochenzement aus einer Kartusche (100),
wobei die Pistole umfasst:
ein Gehäuse (504, 504', 504", 704);
eine Aufnahme (502), die an dem Gehäuse angebracht ist, wobei die Aufnahme ausgeformt ist, um eine Kartusche (100) aufzunehmen, welche Knochenzement enthält;
eine Betätigungsstange (524, 524', 524", 624, 724), die an dem Gehäuse (504, 504', 504", 704) befestigt ist, wobei die Betätigungsstange ein vorderes Ende hat, das dazu ausgeformt ist, gegen einen fest mit der Kartusche (100) verbundenen Kolben (114) zu drücken, und wobei die Betätigungsstange derart beweglich an dem Gehäuse befestigt ist, dass das vordere Ende der Betätigungsstange an dem Kolben der Kartusche anliegen und den Kolben von dem Gehäuse wegdrücken kann; und
eine Verbindungsanordnung (508, 508', 508", 608, 708), wobei die Verbindungsanordnung umfasst:
einen Auslöser (530, 530', 530", 630, 730), der schwenkbar an dem Gehäuse befestigt ist; und ein Betätigungsstangen-Verschiebeelement (528, 528', 528", 628, 662, 786), das innerhalb des Gehäuses vorgesehen ist, wobei das Betätigungsstangen-Verschiebeelement mit dem Auslöser verbunden ist, um durch den Auslöser betätigt zu werden, und dazu ausgebildet ist, durch Betätigung des Auslösers die Betätigungsstange (524, 524', 524", 624, 724) zu erfassen, um die Betätigungsstange gegen den fest mit der Kartusche (100) verbundenen Kolben (114) vorwärts zu bewegen, wobei die Kartusche (100) dabei von der Aufnahme (502) gehalten ist;
wobei die Verbindungsanordnung (508, 508', 508", 608, 708) ferner umfasst:
eine erste Verbindung (532, 532', 532", 632, 732), welche getrennt von dem Auslöser vorgesehen und schwenkbar an dem Gehäuse befestigt ist und dazu ausgebildet ist, das Betätigungsstangen-Verschiebeelement (528, 528', 528", 628, 662, 786) zu erfassen; und
eine zweite Verbindung (536, 536', 536", 636, 746), welche getrennt von dem Auslöser und der ersten Verbindung vorgesehen ist, wobei die zweite Verbindung dazu ausgebildet ist, den Auslöser und die erste Verbindung miteinander zu verbinden, wobei die zweite Verbindung sich durch Schwenken des Auslösers derart bewegt, dass die Bewegung der zweiten Verbindung zu einer Bewegung der ersten Verbindung führt, welche das Betätigungsstangen-Verschiebeelement betätigt;
**dadurch gekennzeichnet, dass** die Verbindungsanordnung (508, 508', 508", 608, 708) ferner umfasst:
eine dritte Verbindung (548, 548', 548", 788), welche getrennt von dem Auslöser, der ersten Verbindung und der zweiten Verbindung vorgesehen ist, wobei die dritte Verbindung dazu ausgebildet ist, den Auslöser mit der Betätigungsstange (524, 524', 524", 624, 724) derart zu verbinden, dass die Bewegung des Auslösers über die dritte Verbindung auf die Betätigungsstange derart übertragen wird, dass die Betätigungsstange sich mit einer höheren Geschwindigkeit vorwärts bewegt, als wenn die erste Verbindung das Betätigungsstangen-Verschiebeelement betätigt, wobei die dritte Verbindung aufweist: eine erfassende Stellung, in welcher die dritte Verbindung den Auslöser mit der Betätigungsstange derart verbindet, dass das Schwenken des Auslösers zu der Vorwärtsbewegung der Betätigungsstange durch die dritte Verbindung führt; und eine gelöste Stellung, in welcher die dritte Verbindung den Auslöser nicht mit der Betätigungsstange verbindet, sodass ein Schwenken des Auslösers nicht dazu führt, dass die dritte Verbindung die Betätigungsstange derart vorwärtsbewegt, dass der Auslöser die erste Verbindung über die zweite Verbindung betätigt, resultierend in einer Vorwärtsbewegung der Betätigungsstange durch das Betätigungsstangen-Verschiebeelement; und
ein Steuerelement (550, 750), das dazu ausgebildet ist, die dritte Verbindung zwischen der erfassenden und gelösten Stellung zu bewegen.

2. Pistole zum Austragen von Knochenzement (500) nach Anspruch 1, wobei:
die Betätigungsstange (724) mit Zähnen (780) ausgebildet ist; und
das Betätigungsstangen-Verschiebeelement eine erste Sperrklinke (786) ist, die dazu ausgeformt ist, die Zähne der Betätigungsstange zu erfassen, wenn die Betätigungsstange in einer Vorwärtsrichtung bewegt wird.

3. Pistole zum Austragen von Knochenzement (500) nach Anspruch 2, wobei die dritte Verbindung eine zweite Sperrklinke (788) ist, welche die Zähne der Betätigungsstange (780) derart erfasst, dass die Verschiebung der zweiten Sperrklinke durch den Auslöser (730) die zweite Sperrklinke dazu veranlasst, die Betätigungsstange in der Vorwärtsrichtung zu bewegen.

4. Pistole zum Austragen von Knochenzement (500) nach Anspruch 2 oder 3, wobei:
die Betätigungsstange (724) derart ausgebildet ist, dass die Zähne (780) sich in einer Längsrichtung über eine erste Oberfläche der Betätigungsstange erstrecken und die Betätigungsstange eine zweite Oberfläche aufweist, die von der ersten Oberfläche bogenförmig beabstandet und glatt ist; und
die Betätigungsstange befestigt ist, um innerhalb des Gehäuses (704) derart drehen zu können, dass entweder die Zähne der ersten Oberfläche der Betätigungsstange oder die zweite Oberfläche der Betätigungsstange in Kontakt mit der ersten Sperrklinke (786) gebracht werden können.

5. Pistole zum Austragen von Knochenzement (500) nach einem der Ansprüche 1, 2 oder 4, wobei die dritte Verbindung (548, 548', 548") der Verbindungsanordnung positioniert ist, um in der erfassenden Stellung das Betätigungsstangen-Verschiebeelement (528, 528', 528", 628, 662, 786) derart zu erfassen, dass die Verschiebung der dritten Verbindung durch den Auslöser (530, 530', 530", 630) dazu führt, dass die dritte Verbindung das Betätigungsstangen-Verschiebeelement derart betätigt, dass das Betätigungsstangen-Verschiebeelement die Betätigungsstange nach vorne bewegt.

6. Pistole zum Austragen von Knochenzement (500) nach Anspruch 1, wobei die dritte Verbindung (788) positioniert ist, um in der erfassenden Stellung die Betätigungsstange (724) derart zu erfassen, dass die dritte Verbindung als eine Komponente fungiert, welche die Betätigungsstange in der Vorwärtsrichtung bewegt, wenn die dritte Verbindung durch den Auslöser (730) betätigt wird.

7. Pistole zum Austragen von Knochenzement (500) nach einem der Ansprüche 1 bis 6, wobei:
der Auslöser (530, 730) derart an dem Gehäuse (504, 704) befestigt ist, dass sich der Auslöser von einer ersten Seite der Betätigungsstange (524, 724) weg und aus dem Gehäuse heraus erstreckt; und
die erste Verbindung (536, 788), die zweite Verbindung (532, 732) und die dritte Verbindung (548, 788) der Verbindungsanordnung (508, 730) derart in dem Gehäuse (504, 704) vorgesehen sind, dass sie benachbart zu einer zweiten Seite der Betätigungsstange angeordnet sind, welche der ersten Seite der Betätigungsstange gegenüberliegt.

8. Pistole zum Austragen von Knochenzement (500) nach einem der Ansprüche 1 bis 7, wobei die Aufnahme (502) umfasst:
zumindest eine Stange (514), die sich aus einem vorderen Ende des Gehäuses (504) heraus erstreckt; und
ein Endstück (510), welches an der zumindest einen Stange befestigt ist, um in einer Vorwärtsrichtung von dem Gehäuse beabstandet zu sein, wobei das Endstück ausgeformt ist, um ein Ende der Kartusche (100) aufzunehmen.

9. Pistole zum Austragen von Knochenzement (500) nach einem der Ansprüche 1 bis 8, wobei:
der Auslöser (530, 530', 730) benachbart zu einem vorderen Ende des Gehäuses (504, 704) angeordnet ist; und
die erste Verbindung (536, 536', 736) schwenkbar an dem Auslöser befestigt ist, um sich in einer Rückwärtsrichtung von dem vorderen Ende des Gehäuses weg zu erstrecken.

10. Pistole zum Austragen von Knochenzement (500) nach einem der Ansprüche 1, 5, 6, 7, 8 oder 9, wobei das Betätigungsstangen-Verschiebeelement ein Greifelement (528, 528', 628, 662) ist, das die Betätigungsstange (524, 524', 524", 624) reibschlüssig erfasst, um die Betätigungsstange vorwärts zu bewegen.

11. Pistole zum Austragen von Knochenzement (500) nach Anspruch 10, wobei das Greifelement (528, 528', 628, 662) eine Öffnung aufweist, durch welche sich die Betätigungsstange (524, 524', 524", 624) erstreckt.

12. Pistole zum Austragen von Knochenzement (500) nach Anspruch 10 oder 11, wobei:
das Greifelement (528, 528', 528", 628) in der Lage ist, sich vorwärts und rückwärts zu bewegen; und
eine Feder (534, 534', 534", 634) sich zwischen dem Gehäuse und dem Greifelement erstreckt, um das Greifelement aus der Erfassung der Betätigungsstange zu bewegen, wenn das Greifelement eine Rückwärtsbewegung vollzieht.

13. Pistole zum Austragen von Knochenzement (500) nach einem der Ansprüche 10, 11 oder 12, ferner umfassend einen Lösestift (558), der beweglich an dem Gehäuse (504) befestigt ist, wobei der Lösestift aufweist:
eine gelöste Stellung, in welche der Lösestift von dem Greifelement (528) beabstandet ist; und
eine erfassende Stellung, in welcher der Lösestift das Greifelement (528) erfasst und das Greifelement derart verschiebt, dass das Greifelement sich aus der Erfassung mit der Betätigungsstange bewegt.

14. Pistole zum Austragen von Knochenzement (500) nach einem der Ansprüche 10, 11, 12 oder 13, wobei das Betätigungsstangen-Verschiebeelement eine Mehrzahl von Greifplatten (528 und 562, 628 und 662) umfasst, die gegen die Betätigungsstange (524, 624) drücken.

15. Pistole zum Austragen von Knochenzement (500) nach einem der Ansprüche 1 bis 14, wobei die dritte Verbindung (548, 788) derart schwenkbar an der zweiten Verbindung (536, 736) befestigt ist, dass die Verschiebung der zweiten Verbindung durch den Auslöser (530, 730) zu einer Verschiebung der dritten Verbindung durch die zweite Verbindung führt.

16. Pistole zum Austragen von Knochenzement (500) nach einem der Ansprüche 1 bis 14, wobei die dritte Verbindung (548', 548") schwenkbar mit dem Betätigungsstangen-Verschiebeelement (528', 528") verbunden ist.

17. Pistole zum Austragen von Knochenzement (500) nach Anspruch 16, wobei der Auslöser (530'), das Betätigungsstangen-Verschiebeelement (528') und die dritte Verbindung (548') gemeinsam derart an dem Gehäuse (504) befestigt sind, dass, in der erfassenden Stellung der dritten Verbindung, sich die dritte Verbindung in einer Vorwärtsrichtung von dem Betätigungsstangen-Verschiebeelement erstreckt.

18. Pistole zum Austragen von Knochenzement (500) nach Anspruch 16, wobei der Auslöser (530"), das Betätigungsstangen-Verschiebeelement (528") und die dritte Verbindung (548") gemeinsam derart an dem Gehäuse (504) befestigt sind, dass, in der erfassenden Stellung der dritten Verbindung, sich die dritte Verbindung in einer Rückwärtsrichtung von dem Betätigungsstangen-Verschiebeelement erstreckt.

## Revendications

1. Pistolet (500) pour administrer du ciment osseux depuis une cartouche (100), ledit pistolet incluant :
un logement (504, 504', 504", 704) ;
un berceau (502) fixé au logement, le berceau formé pour supporter une cartouche (100) contenant du ciment osseux ;
une tige d'entraînement (524, 524', 524", 624, 724) montée sur le logement (504, 504', 504", 704), la tige d'entraînement ayant une extrémité avant formée pour agir contre un piston (114) intégré avec la cartouche (100) et la tige d'entraînement étant montée mobile sur le logement de telle manière que l'extrémité avant de la tige d'entraînement peut venir buter sur et pousser le piston de cartouche à l'opposé du logement ; et
un ensemble de liaison (508, 508', 508", 608, 708), l'ensemble de liaison incluant : une gâchette (530, 530', 530", 630, 730) montée pivotante sur le logement ; et un élément (528, 528', 528", 628, 662, 786) d'avancement de tige d'entraînement disposé dans le logement, l'élément d'avancement de tige d'entraînement étant connecté à la gâchette pour être actionné par la gâchette et adapté à, à l'actionnement de la gâchette, engager la tige d'entraînement (524, 524', 524", 624, 724) de manière à faire avancer la tige d'entraînement contre le piston (114) intégré avec la cartouche (100) maintenue par le berceau (502) ;
dans lequel l'ensemble de liaison (508, 508', 508", 608, 708), inclut en outre :
une première liaison (532, 532', 532", 632, 732) séparée de ladite gâchette, ladite première liaison étant monté pivotante sur ledit logement et adaptée à engager l'élément (528, 528', 528", 628, 786) d'avancement de tige d'entraînement ; et
une deuxième liaison (536, 536', 536", 636, 746) séparée de la gâchette et de ladite première liaison, ladite deuxième liaison adaptée à interconnecter ladite gâchette et ladite première liaison, dans lequel ladite deuxième liaison se déplace avec le pivotement de ladite gâchette de telle manière que le mouvement de ladite deuxième liaison résulte en un mouvement de la première liaison qui actionne l'élément d'avancement de tige d'entraînement ;
**caractérisé en ce que** l'ensemble de liaison (508, 508', 508", 608, 708), inclut en outre :
une troisième liaison (548, 548', 548", 788) séparée de la gâchette, de ladite première liaison, et de ladite deuxième liaison, ladite troisième liaison adaptée à relier la gâchette à la tige d'entraînement (524, 524', 524", 624, 724) de telle manière que le mouvement de la gâchette est transféré par l'intermédiaire de ladite troisième liaison à la tige d'entraînement de telle manière que ladite tige d'entraînement avance à une vitesse plus grande que lorsque ladite première liaison actionne ledit élément d'avancement de tige d'entraînement, ladite troisième liaison ayant : une position engagée dans laquelle ladite troisième liaison connecte la gâchette à ladite tige d'entraînement de telle manière que le pivotement de ladite gâchette résulte en l'avancement de ladite tige d'entraînement par ladite troisième liaison ; et une position désengagée dans laquelle ladite troisième liaison ne connecte pas la gâchette à ladite tige d'entraînement de telle manière que le pivotement de ladite gâchette ne résulte pas **en ce que** ladite troisième liaison fasse avancer ladite tige d'entraînement de telle manière que ladite gâchette actionne la première liaison par l'intermédiaire de la deuxième liaison, résultant en ledit élément d'avancement de tige d'entraînement faisant avancer ladite tige d'entraînement ; et
un élément (550, 750) de commande adapté à déplacer ladite troisième liaison entre les positions engagée et désengagée.

2. Pistolet (500) d'administration de ciment osseux selon la revendication 1, dans lequel :
la tige d'entraînement (724) est formée avec des dents (780) ; et
l'élément d'avancement de tige d'entraînement est un premier cliquet (786) formé pour engager lesdites dents de tige d'entraînement lors du déplacement de ladite tige d'entraînement dans un sens vers l'avant.

3. Pistolet (500) d'administration de ciment osseux selon la revendication 2, dans lequel ladite troisième liaison est un deuxième cliquet (788) qui engage lesdites dents (780) de tige d'entraînement de telle manière que le déplacement dudit deuxième cliquet par la gâchette (730) fait que ledit deuxième cliquet déplace ladite tige d'entraînement dans le sens vers l'avant.

4. Pistolet (500) d'administration de ciment osseux selon les revendications 2 ou 3, dans lequel :
la tige d'entraînement (724) est formée de telle manière que lesdites dents (780) s'étendent longitudinalement par-dessus une première surface de ladite tige d'entraînement et ladite tige d'entraînement est formée avec une deuxième surface qui est espacée en arc de cercle de la première surface et qui est lisse ; et
la tige d'entraînement est montée pour tourner à l'intérieur dudit logement (704) de telle manière que l'une ou l'autre desdites dents de la première surface de tige d'entraînement ou de la deuxième surface peut/peuvent être placée(s) en contact avec ledit premier cliquet (786).

5. Pistolet (500) d'administration de ciment osseux selon l'une quelconque des revendications 1, 2 ou 4, dans lequel ladite troisième liaison (548, 548', 548") d'ensemble de liaison est positionnée de manière à, lorsqu'elle est dans la position engagée, engager l'élément (528, 528', 528", 628) d'avancement de tige d'entraînement de telle manière que le déplacement de ladite troisième liaison par la gâchette (530, 530', 530", 630) résulte en ladite troisième liaison actionnant ledit élément d'avancement de tige d'entraînement de telle manière que ledit élément d'avancement de tige d'entraînement fait avancer ladite tige d'entraînement.

6. Pistolet (500) d'administration de ciment osseux selon la revendication 1, dans lequel ladite troisième liaison (788) est positionnée de manière à, lorsqu'elle est dans la position engagée, engager la tige d'entraînement (724) de telle manière que ladite troisième liaison fonctionne comme un composant qui, lorsque ladite troisième liaison est actionnée par la gâchette (730), déplace ladite tige d'entraînement dans le sens vers l'avant.

7. Pistolet (500) d'administration de ciment osseux selon l'une quelconque des revendications 1 à 6, dans lequel :
la gâchette (530, 730) est montée sur le logement (504, 704) de telle manière que la gâchette s'étend à l'opposé d'un premier côté de la tige d'entraînement (524, 724) et hors dudit logement ;
ladite première liaison (536, 788), ladite deuxième liaison (532, 732) et ladite troisième liaison (548, 788) de l'ensemble de liaison (508, 730) sont disposées dans ledit logement (504, 704) de manière à être situées adjacentes à un deuxième côté de ladite tige d'entraînement qui est opposé au premier côté de ladite tige d'entraînement.

8. Pistolet (500) d'administration de ciment osseux selon l'une quelconque des revendications 1 à 7, dans lequel le berceau (502) inclut :
au moins une barre (514) qui s'étend vers l'avant depuis une extrémité avant dudit logement (504) ; et
une plaque d'extrémité (510) montée sur ladite au moins une barre de manière à être espacée vers l'avant par rapport audit logement, ladite plaque d'extrémité formée pour recevoir une extrémité de la cartouche (100).

9. Pistolet d'administration de ciment osseux selon l'une quelconque des revendications 1 à 8, dans lequel :
la gâchette (530, 530', 730) est située adjacente à une extrémité avant du logement (504, 704) ; et
la première liaison (536, 536', 736) est montée pivotante sur la gâchette pour s'étendre vers l'arrière à l'opposé de l'extrémité avant dudit logement.

10. Pistolet (500) d'administration de ciment osseux selon l'une quelconque des revendications 1, 5, 6, 7, 8 ou 9, dans lequel l'élément d'avancement de tige d'entraînement est un élément (528, 528', 628, 662) de préhension qui engage par frottement la tige d'entraînement (524, 524', 524", 624) pour faire avancer la tige d'entraînement.

11. Pistolet (500) d'administration de ciment osseux selon la revendication 10, dans lequel ledit élément (528, 528', 528", 628) de préhension est formé avec une ouverture à travers laquelle ladite tige d'entraînement (524, 524', 524", 624) s'étend.

12. Pistolet (500) d'administration de ciment osseux selon les revendications 10 ou 11, dans lequel :
ledit élément (528, 528', 528", 628) de préhension est apte à la fois à un mouvement vers l'avant et vers l'arrière ; et
un ressort (534, 534', 534", 634) s'étend entre ledit logement et ledit élément de préhension de manière à déplacer ledit élément de préhension hors d'engagement avec ladite tige d'entraînement lorsque ledit élément de préhension subit un mouvement vers l'arrière.

13. Pistolet (500) d'administration de ciment osseux selon l'une quelconque des revendications 10, 11 ou 12, incluant en outre une goupille de déverrouillage (558) qui est montée mobile sur ledit logement (504), ladite goupille de déverrouillage ayant :
une position désengagée dans laquelle ladite goupille de déverrouillage est espacée dudit élément (528) de préhension ; et
une position engagée dans laquelle ladite goupille de déverrouillage engage et déplace ledit élément (528) de préhension de telle manière que ledit élément de préhension sort d'engagement avec ladite tige d'entraînement.

14. Pistolet (500) d'administration de ciment osseux selon l'une quelconque des revendications 10, 11, 12 ou 13, dans lequel l'élément d'avancement de tige d'entraînement inclut une pluralité de plaques (528 et 562, 628 et 662) de préhension qui portent contre la tige d'entraînement (524, 624).

15. Pistolet (500) d'administration de ciment osseux selon l'une quelconque des revendications 1 à 14, dans lequel ladite troisième liaison (548, 788) est montée pivotante sur ladite deuxième liaison (536, 736) de telle manière que le déplacement de ladite deuxième liaison par ladite gâchette (530, 730) résulte en le déplacement de ladite troisième liaison par ladite deuxième liaison.

16. Pistolet (500) d'administration de ciment osseux selon l'une quelconque des revendications 1 à 14, dans lequel ladite troisième liaison (548', 548") est connectée pivotante à l'élément (528', 528") d'avancement de tige d'entraînement.

17. Pistolet (500) d'administration de ciment osseux selon la revendication 16, dans lequel la gâchette (530'), l'élément (528') d'avancement de tige d'entraînement et ladite troisième liaison (548') sont collectivement montés sur le logement (504) de telle manière que, lorsque ladite troisième liaison est dans la position engagée, ladite troisième liaison s'étend vers l'avant dudit élément d'avancement de tige d'entraînement.

18. Pistolet (500) d'administration de ciment osseux selon la revendication 16, dans lequel la gâchette (530"), l'élément (528") d'avancement de tige d'entraînement et ladite troisième liaison (548") sont collectivement montés sur le logement (504) de telle manière que, lorsque ladite troisième liaison est dans la position engagée, ladite troisième liaison s'étend vers l'arrière dudit élément d'avancement de tige d'entraînement.
